# EUROPÄISCHE PATENTANMELDUNG

(11) **EP 4 710 955 A1**
(43) Veröffentlichungstag der Anmeldung: **18.03.2026**
(21) Anmeldenummer: 24315421.8
(22) Anmeldetag: 16.09.2024
(51) Int. Cl.: A61L 15/22, A61L 15/26, A61L 15/46

(54) **SILIKONHALTIGE WUNDKONTAKTSCHICHT MIT INFEKTIONSHEMMENDEN EIGENSCHAFTEN**

(71) Anmelder: Paul Hartmann AG, 89522 Heidenheim (DE)
(72) Erfinder: Kettel, Markus, 89522 Heidenheim (DE); Karl, Michael Maximilian, 89522 Heidenheim (DE); Vrana, Nihal Engin, 67000 Strasbourg (FR); Skander, Hathroubi, 67000 Strasbourg (FR)
(74) Vertreter: Paul Hartmann AG Patents & Licensing

(57) **Zusammenfassung**

Die vorliegende Erfindung betrifft eine silikonhaltige, antimikrobielle Wundkontaktschicht, die wundseitig eine teilweise oder vollständige antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst. Weiterhin umfasst die Erfindung absorbierende Wundauflagen, die die genannte Wundkontaktschicht beinhalten, sowie Verfahren zur Herstellung der Wundkontaktschicht.

## Beschreibung

### Technisches Gebiet der Erfindung

Die vorliegende Erfindung betrifft die Wundbehandlung, insbesondere die Behandlung schmerzhafter, stark exsudierender und/oder infizierter Wunden. Diese Umstände treten insbesondere bei chronischen Wunden auf.

### Hintergrund der Erfindung

Chronische Wunden stellen in der modernen Medizin nach wie vor ein Problem dar. Gerade bei älteren Menschen und Risikopatienten wie beispielsweise Diabetikern besteht ein erhöhtes Risiko, dass Gewebeschäden nicht oder nicht vollständig abheilen. Der Wundheilungsprozess ist in einem solchen Fall aus unterschiedlichen Gründen gestört und es besteht ein fortwährender Defekt der Hautbarriere. Diese Wunden neigen zu einer übermäßigen Produktion von Wundexsudat. Um eine Mazeration der Wundränder durch das Wundexsudat zu vermeiden, ist es üblich absorbierende Wundauflagen zu verwenden und diese zu wechseln, sobald deren Absorptionskapazität erschöpft ist. Häufig geht der Verbandswechsel mit Schmerzen für die Patienten einher. Zudem besteht die Gefahr, dass die absorbierenden Wundauflagen an dem Wundbett anhaften (z.B. durch Festkleben oder Antrocknen) und es beim Entfernen des Verbandsmaterials zu Blutungen kommt.

Mit fortwährender Dauer der Gewebeexposition der chronischen Wunden erhöht sich zunehmend das Risiko einer Infektion. Tritt eine solche Infektion ein, verschlechtert sich die Prognose weiter. Da die körpereigenen Stoffwechselprozesse im Bereich der chronischen Wunde beeinträchtigt sind und eine reguläre Immunantwort nicht oder nicht vollständig erfolgt, kann es in der Folge zu einer Vermehrung der Erreger an der Infektionsstelle kommen. Im weiteren Verlauf bildet sich häufig ein Biofilm, bei dem bakterielle Erreger sich zu einer Lebensgemeinschaft vereinen, die erhöhte Resistenz gegenüber Bioziden und Antibiotika zeigt und daher die weitere Behandlung außerordentlich erschwert. Dieses Stadium erhöht die Wahrscheinlichkeit für weitere Komplikationen wie beispielsweise Nekrosenbildung oder Sepsis.

Aus dem Stand der Technik sind sowohl absorbierende Wundauflagen als auch atraumatische Wundauflagen und solche mit antibiotischer Wirksamkeit bekannt. Letztere beruhen in der Regel auf der Verwendung körperfremder antimikrobieller Wirkstoffe. Derartige antibiotische Wundauflagen zeigen zwar eine Wirkung gegen pathogene Mikroorganismen, haben jedoch die nachteilige Eigenschaft auch körpereigene Zellen zu beeinträchtigen. Mittels *in vitro* Assays konnte eine erhöhte Zytotoxizität für tierische Zellen nachgewiesen werden. Die betroffenen Zellen werden gestresst und ihre Vitalwerte sinken ab. Bei Testkonzentrationen, die den Realbedingungen nahekommen, stirbt für gewöhnlich ein Teil der im Assay befindlichen Zellen ab.

Absorbierende Wundauflagen können teilweise erhebliche Mengen an Wundexsudat aufnehmen, eignen sich jedoch nicht für alle Arten von Wunden gleichermaßen. Bei Wunden die wenig oder gar kein Wundexsudat produzieren, kann durch Einsatz von Absorptionsmaterial die Ausbildung eines vorteilhaften feuchten Wundmilieus verhindert werden. Zudem geht die Produktion derartiger Wundauflagen mit hohem Materialvolumen und entsprechenden Kosten einher.

Die WO 2020/126898 A1 beschreibt eine atraumatische Wundauflage mit Superabsorber. Die Wundauflage verfügt jedoch über keine antimikrobiellen Mechanismen.

Die EP 1 755 569 B9 beschreibt eine Wundauflage mit Salbe, die zusätzlich ein antibakterielles Metall wie beispielsweise Silber enthält. Allerdings kann die Wirkung von Silber mit deutlich messbarer Zytotoxizität einhergehen..

Die EP 3 452 118 B1 beschreibt antimikrobielle Beschichtungen, die Hyaluronsäure und ein Polypeptid enthalten, wobei die Säure und das Peptid nicht durchmischt vorliegen. Spezifische Ausgestaltungen von Wundauflagen werden nicht genannt.

Die EP 2 371 335 B1 beschreibt eine antibakterielle Wundauflage mit dem Wirkstoff Polyhexamethylenbiguanid (PHMB). Jüngste Untersuchungen haben jedoch gezeigt, dass PHMB problematischer ist, als ursprünglich angenommen wurde. So wurden toxische Effekt im *in vitro* Assay bei Humanzellen festgestellt (Medical mycology, 2017, 55. Jg., Nr. 3, S. 334-343) und auch *in vivo* Versuche an Ratten zeigten ein ernstzunehmendes Schädigungspotential (Interdisciplinary Toxicology, 2015, 8. Jg., Nr. 4, S. 193-202).

Folglich besteht ein Bedarf nach einem Mittel zur Wundversorgung, das einen schmerzfreien und atraumatischen Verbandswechsel ermöglicht, antimikrobielle und infektionshemmende Eigenschaften hat und darüber hinaus derart ausgestaltet werden kann, dass eine Versorgung auch stark exsudierender Wunden ermöglicht wird. Um dem klinischen Alltag gerecht zu werden, sollte das Mittel der Wahl zudem unmittelbar einsetzbar sein, bei längerer Lagerung über eine ausreichende Stabilität verfügen und beständig gegenüber möglichen Temperaturschwankungen sein.

### Zusammenfassung der Erfindung

Die oben genannte Aufgabe wird gelöst durch eine silikonhaltige, antimikrobielle Wundkontaktschicht, die wundseitig eine teilweise oder vollständige antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst. Die Wundkontaktschicht kann zudem mit absorbierenden Materialien kombiniert werden, um ein angemessenes Exsudatmanagement zu ermöglichen.

Die erfindungsgemäße Wundkontaktschicht hat hervorragende atraumatische Eigenschaften, d.h. sie verbindet sich nicht mit der Wunde oder Wundbestandteilen. Weder kann Gewebe in die Wundkontaktschicht einwachsen, noch kann sie am Wundbett ankleben. Der für gewöhnlich notwendige Einsatz von Salbe zum Erreichen der atraumatischen Eigenschaften ist bei der vorliegenden Erfindung nicht notwendig, da diese Rolle von der antimikrobiellen Beschichtung in Kombination mit Silikon erfüllt wird. Schließlich ist die Wundkontaktschicht aufgrund ihrer antimikrobiellen Beschichtung gegen humanpathogene Bakterien wirksam. Zudem kann die Wundkontaktschicht Bestandteil einer Wundauflage sein. Die Wundauflage kann dabei für die Behandlung unterschiedlicher Wundarten spezifisch zusammengestellt werden, um die bestmögliche Versorgung zu gewährleisten.

Die erfindungsgemäße Wundkontaktschicht kann auch dann zum Einsatz kommen, wenn sich in der zu behandelnden Wunde antibiotikaresistente Bakterien befinden. Während Antibiotika üblicherweise organische Verbindungen sind, die von resistenten Bakterien beispielsweise abgebaut werden, ist ein derartiges Auftreten von Resistenzen im Hinblick auf die antimikrobielle Beschichtung der vorliegenden Erfindung nicht beobachtet worden.

Weiterhin weist die erfindungsgemäße Wundkontaktschicht wundheilungsfördernde Eigenschaften auf. Dies ergibt sich durch die feuchtigkeitsregulierenden Eigenschaften der Hyaluronsäure, die auch *in vivo* im Bereich der extrazellulären Matrix zu finden ist.

Nachfolgend wird erläutert, wie die Wundkontaktschicht und die zugehörige Beschichtung strukturell und chemisch beschaffen sein können, um in der Praxis den größtmöglichen Nutzen zu erbringen.

### Detaillierte Beschreibung der Erfindung

Der Begriff "medizinisch akzeptables Material" im Sinne der Erfindung ist eine ungiftige, fusselfreie und stabile Substanz (z.B. ein Substrat), die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "Vliesmaterial" meint eine Schicht von miteinander zusammenhängenden Fasern, die nicht gewebt sind und in der Regel nicht nach einem sich wiederholendem Muster angeordnet sind.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers (z.B. eines Netzes, Gitters oder Trägermaterials) mittels einer Substanz, die sich von der Struktur des Festkörpers unterscheidet, zumindest teilweise bedeckt bzw. überlagert wird. Insbesondere kann die Beschichtung faserlos und / oder gelartig sein. Somit kann es sich bei der Beschichtung um ein Gel, insbesondere ein hydrophiles Gel handeln.

Der Ausdruck "atraumatisch" meint, dass ein Wundversorgungsprodukt sich nicht mit der Wunde verbindet oder mit der Wunde verklebt, also beispielsweise nicht in der Wunde eintrocknet oder mit dieser verwächst und dass eine schmerzfreie Entfernung des Produktes ohne Störung des Heilungsprozesses möglich ist.

Der Ausdruck "antimikrobielle Inhaltsstoffe" meint Hyaluronsäure und ein oder mehrere Polypeptide, bei denen es sich um Polyarginin und / oder Polylysin und / oder Polyornithin handelt.

Der Ausdruck "antimikrobiell" meint im weitesten Sinne, dass ein Wirkstoff, Wirkstoffgemisch oder ein damit behandelter Artikel (z.B. eine Wundkontaktschicht) in der Lage ist, die Vermehrung von Mikroorganismen zu hemmen oder aufzuhalten oder die Anzahl vitaler Mikroorganismen zu reduzieren. Im engeren Sinne ist gemeint, dass ein solcher Artikel in der Lage ist, in einem Test nach ISO 20743:2021 die Anzahl der CFU des *Pseudomonas aeruginosa* Stammes mit der Hinterlegungsnummer ATTC 27853 und / oder des *Staphylococcus aureus* Stammes mit der Hinterlegungsnummer ATTC 25923 um mindestens drei Logarithmusstufen, bevorzugt um mindestens vier Logarithmusstufen, besonders bevorzugt um mindestens fünf Logarithmusstufen und ganz besonders bevorzugt um mindestens sechs Logarithmusstufen im Vergleich zu einem baugleichen Artikel ohne Wirkstoff zu reduzieren. Darüber hinaus schließt der Ausdruck "antimikrobiell" den Ausdruck "antibakteriell" mit ein.

Die Ausdrücke "Aminosäure" und "Aminosäuren" beziehen sich - sofern nicht anders angegeben - auf die Verbindungen Arginin, Lysin und / oder Ornithin, welche allesamt zu den positiv geladenen Aminosäuren zählen. Dies schließt insbesondere die L-Enantiomere der genannten Aminosäuren ein.

Die Ausdrücke "Polypeptid" und "antimikrobielles Polypeptid" meinen vorliegend Polyarginin, Polylysin und / oder Polyornithin und beziehen sich auf Peptidverbindungen, die mindestens 10, bevorzugt mindestens 20, besonders bevorzugt mindestens 30 Untereinheiten in Form von verbundenen Aminosäuren umfassen. Im Rahmen der Erfindung können die Aminosäuren innerhalb eines solchen Polypeptids allesamt identisch sein (selbe Aminosäure). Alternativ können Mischungen aus zwei oder drei der zuvor genannten Aminosäuren innerhalb eines Polypeptids vorliegen. Alle derartigen Polypeptide haben eine positive Nettoladung. Dem Fachmann ist bekannt, dass sich für die Aminosäuren am C-terminalen sowie N-terminalen Ende naturgemäß strukturelle Unterschiede ergeben, da diese Positionen die jeweiligen Kettenenden darstellen.

Der Ausdruck "Polylysin" umfasst die stereochemische Variante α-Poly-L-Lysin und / oder ε-Poly-L-Lysin.

Die Ausdrücke "abwechseln" bzw. "abwechselnd" sowie "alternierend" meinen, dass auf eine erste Lage enthaltend Hyaluronsäure eine zweite Lage enthaltend das Polypeptid oder die Polypeptide folgt. Eine optionale dritte Lage würde folglich wieder Hyaluronsäure enthalten. Auf diese Wiese wechseln sich die Lagen in ihrer Nettoladung ab und bilden aufgrund ihrer Anziehungskräfte eine stabile Beschichtung aus. Alternativ kann in diesem Sinne natürlich auch auf eine erste Lage enthaltend das Polypeptid oder die Polypeptide eine zweite Lage enthaltend die Hyaluronsäure usw. folgen.

Der Ausdruck "proximal" bedeutet, dass ein Element im Einsatz zur Wunde oder Haut hin ausgerichtet bzw. wundzugewandt ist.

Der Ausdruck "distal" bedeutet, dass ein Element im Einsatz von der Wunde oder Haut weg zeigt bzw. wundabgewandt ist.

Die vorliegende Erfindung betrifft eine silikonhaltige, antimikrobielle Wundkontaktschicht. Die Wundkontaktschicht kann allein verwendet werden oder nach dem Anbringen auf eine Wunde bei Bedarf durch einen Sekundärverband überlagert werden oder mittels Sekundärverband, Klebefolie, Klebestreifen oder anderer Fixiermittel am Ort der Wunde befestigt werden. Die antimikrobielle Beschichtung der Wundkontaktschicht ist im Einsatz zur Wunde hin ausgerichtet.

Vorteilhaft ist, dass die Wundkontaktschicht sowohl mittels der enthaltenen Beschichtung als auch mittels des Silikons atraumatisch ist und nicht mit der Wunde verklebt. Dies ermöglicht ein schmerzfreies und unkompliziertes Ablösen des Verbandsmaterials. Gleichzeitig erlaubt die Beschichtung eine leichte Initialhaftung, und zwar insbesondere an trockenem Gewebe. Dies erleichtert das Anbringen der beschichteten Wundkontaktschicht im Bereich der Wunde, denn in den meisten Fällen muss das Material nicht bis zur Applikation eines Fixiermittels an der Wundstelle festgehalten werden, sondern haftet zunächst von allein, so dass dem Anwender beide Hände für die Vorbereitung eines Fixiermittels (z.B. Klebestreifen oder Klebefolie) zur Verfügung stehen. Die Wundkontaktschicht kann auch Bestandteil einer absorbierende Wundauflage sein, welche optional als sogenannter Inselverband mit umlaufendem Kleberand ausgestaltet sein kann. Dieser Kleberand kann mittels hautkompatibler Klebstoffe bereitgestellt werden, wobei Acrylkleber, Silikonkleber und synthetischer Kautschuk zu den gebräuchlichsten Mitteln gehören. Diese haben gleichzeitig den Vorteil restlos ablösbar zu sein.

Die erfindungsgemäße Wundkontaktschicht eignet sich für akute, chronische sowie für infizierte Wunden. Insbesondere chronische Wunden, die regelmäße und häufige Verbandswechsel erfordern, können hervorragend versorgt werden. Beispiele für chronische Wunden sind Dekubitus, Ulcus Cruris, ischämische Wunden, diabetische Wunden und eiternde Wunden.

in der Praxis treten häufig Mischformen dieser Wunden auf, z.B. eiternde Wunden bei Ulcus Cruris. In solchen Fällen zeigen sich die vorteilhaften Eigenschaften der erfindungsgemäßen Wundkontaktschicht besonders deutlich. Daneben hat die antimikrobielle Beschichtung auch bei nicht infizierten Wunden den Vorteil, eine mögliche spätere Besiedelung mit Erregern zu hemmen oder zu unterbinden.

Die erfindungsgemäße Wundkontaktschicht umfasst ein Silikon oder silikonhaltiges Material, welches zumindest wundseitig - also proximal - eine teilweise oder vollständige antimikrobielle Beschichtung aufweist, die antimikrobielle Inhaltsstoffe enthält. Die antimikrobielle Beschichtung ist insbesondere antibakteriell. Weitere Bestandteile können der Wundkontaktschicht bei Bedarf hinzugefügt werden, um eine Wundauflage zu erhalten. Dies wird an anderer Stelle näher erläutert.

Im Rahmen der vorliegenden Erfindung kann vorgesehen sein, dass sich die Beschichtung ausschließlich auf der proximalen Seite der Wundkontaktschicht befindet und damit auf derjenigen Seite, welche in Benutzung der Wunde zugewandt ist. Diese Ausführung hat den ökonomischen Vorteil, dass Beschichtungsmaterial eingespart wird, ohne dass die Versorgung der Wunde darunter leidet. Daneben können auch beide Seiten (distal und proximal) die Beschichtung tragen, was den Vorteil hat, dass der Anwender nicht darauf achten muss, welche Seite der Wundkontaktschicht auf die Wunde aufzulegen ist. Daneben bietet eine beidseitig beschichtete Wundkontaktschicht auch Vorteile beim Ausfüllen von tiefen Wunden und Gewebedefekten, welche gelegentlich als Kavitäten bezeichnet werden.

in der Regel ist die Wundkontaktschicht flach bzw. planar, so dass sie eine im Wesentlichen einheitliche Dicke aufweist und weder die proximale noch die distale Seite nennenswerte (z.B. makroskopische) Unebenheiten oder Erhebungen aufweisen. Geringfügige produktionsbedingte Toleranzen sind hierbei zu vernachlässigen, so dass im Sinne der Erfindung eine einheitliche Dicke auch bei Abweichungen von +/- 5 % als gegeben gelten soll. Eine flach ausgebildete Wundkontaktschicht kann in der Draufsicht eine rechteckige, quadratische, ovale oder runde Form haben, wobei ovale oder runde Formen sich besonders für Wunden an Gelenken eignen und rechteckige oder quadratische Formen eine effizientere Ausbeute von Bahnenmaterial (z.B. mittels Schneiden oder Stanzen) ermöglichen und auch eine bessere Nutzung von Lagerplatz erlauben.

Die Wundkontaktschicht kann Perforationen aufweisen. Hierbei handelt es sich um durchgehende Öffnungen, die sich durch die gesamte Dicke der Wundkontaktschicht (proximal-distale Ausrichtung) erstrecken. Die Öffnungen ermöglichen einen Durchfluss von Wundsekret, so dass ein Sekretstau vermieden wird. Die Öffnungen können unterschiedliche Formen aufweisen. Bevorzugt sind kreisförmige oder eckige Öffnungen. Durch den Einsatz von eckigen Öffnungen erhält man eine Wundkontaktfläche in Form eines Wundkontaktgitters.

Bei der antimikrobiellen Beschichtung handelt es sich bevorzugt um eine gleichmäßige oder im Wesentlichen gleichmäßige Verteilung der Beschichtungsmasse, bei der jeder beschichtete Bereich der Wundkontaktschicht mit derselben oder im Wesentlichen derselben Menge an Beschichtung ausgestattet ist.

Die erfindungsgemäße Beschichtung enthält mindestens die hierin beschriebene Hyaluronsäure und das hierin beschriebene Polypeptid bzw. die Polypeptide. Weitere Verbindungen oder strukturelle Komponenten können ebenfalls Teil der Beschichtung sein oder mit dieser kombiniert werden. Diese weiteren Verbindungen oder strukturellen Komponenten können flüssig oder fest sein. Darüber hinaus können sie positiv geladen, negativ geladen oder ladungsneutral sein.

In diesem Sinne kann die Beschichtung eine polare Flüssigkeit enthalten. Bei der polaren Flüssigkeit kann es sich um Wasser handeln. Das Wasser kann als destilliertes oder deionisiertes Wasser vorliegen. Bevorzugt liegt die polare Flüssigkeit (z.B. Wasser) als wässrige Pufferlösung vor. Beispiele für wässrige Puffer sind Citratpuffer, Ringerlösung, TRIS-Puffer, TE-Puffer, TBS-Puffer und TBS-T-Puffer. Bevorzugt handelt es sich bei dem Puffer um Tris-NaCl-Puffer. Die Konzentration des Puffers im Lösungsmittel (z. B. Wasser) kann beispielsweise 5 mmol bis 300 mmol betragen, bevorzugt beträgt die Konzentration 10 mmol bis 200 mmol. Im Falle von Tris-NaCl-Puffer kann die Konzentration von Tris beispielsweise 5 bis 300 mmol und die Konzentration von NaCl 10 mmol bis 300 mmol betragen. Alternativ kann die Konzentration des Puffers so gewählt werden, dass die gepufferte Lösung einen pH von 6,8 bis 7,8, bevorzugt 7,2 bis 7,6 hat. Diese pH-Werte beziehen sich zunächst auf die Lösung vor ihrer Vermengung mit anderen Bestandteilen der Beschichtung. Allerdings sind diese Werte auch auf die fertige Beschichtung im finalen Produkt - also der erfindungsgemäßen Wundkontaktschicht - anwendbar, und zwar sowohl vor als auch nach einer optionalen Trocknung.

Die Masse der bereits erwähnten Puffersubstanzen in Form einer Base oder einer Säure sowie einem geeigneten Salz dieser Base oder Säure kann ein Flächengewicht in der Beschichtung von beispielsweise 50 ng bis 1.000 ng / cm² Wundkontaktschicht ausmachen, wobei die Öffnungen (Perforationen) in der Wundkontaktschicht dabei unberücksichtigt bleiben. Bevorzugt haben die Puffersubstanzen in der Beschichtung ein Flächengewicht von 100 ng bis 500 ng / cm² Wundkontaktschicht, besonders bevorzugt ein Flächengewicht von 120 ng bis 300 ng / cm² Wundkontaktschicht. Bevorzugt enthält die Puffersubstanz eine Base, besonders bevorzugt handelt es sich um Tris und ganz besonders bevorzugt wird die Base Tris kombiniert mit dem Salz NaCl.

Die Beschichtung kann einen Konservierungsstoff oder einen Stabilisator in einer Menge von 0,1 bis 2 Gew.-% enthalten. Beispiele für geeignete Konservierungsstoffe sind beispielsweise Benzoesäure, Sorbinsäure oder Parabene. Beispiele für geeignete Stabilisatoren sind Ascorbyl Palmitate und Tocopherol. Da die Wundkontaktschicht mitsamt Beschichtung in der Regel vor der Verwendung sterilisiert wird, kann in den meisten Fällen auf die Verwendung von Konservierungsstoffen verzichtet werden, um den Produktionsaufwand gering zu halten. Vorzugsweise kann auch vorgesehen sein, dass die Beschichtung generell keine Konservierungsstoffe und / oder Stabilisatoren enthält, um die Wahrscheinlichkeit für das Auftreten von Allergien und Unverträglichkeitsreaktionen zu reduzieren. In diesem Sinne kann die erfindungsgemäße Beschichtung und die damit ausgestattete Wundkontaktschicht antiallergen sein.

Der Gehalt an polaren Flüssigkeiten, insbesondere an Wasser, in der Beschichtung kann 0,1 bis 50 Gew.-% betragen. In manchen Fällen (z.B., wenn die Beschichtung als Gel vorliegen soll) können auch mehr als 50 Gew.-% an polaren Flüssigkeiten gewünscht und sinnvoll sein. Bevorzugt enthält die Beschichtung 1 bis 45 Gew.-% polare Flüssigkeiten, besser 3 bis 40 Gew.-% polare Flüssigkeiten und am besten enthält die Beschichtung 5 bis 35 Gew.-% polare Flüssigkeiten. Diese Konzentrationen können sich auf den finalen Gehalt im fertigen Produkt nach aktiver oder passiver Trocknung beziehen. Es können zwei oder mehr verschiedene polare Flüssigkeiten in der Beschichtung vorhanden sein. Beispiele für mögliche Kombinationen aus polaren Flüssigkeiten sind Wasser und Ethanol oder Wasser und Glycerin. Darüber hinaus kann es sich bei der polaren Flüssigkeit um eine flüssige Pufferlösung wie beispielsweise Tris-NaCl-Puffer handeln. Die polare Flüssigkeit bzw. die Pufferlösung können hierbei einen pH von 6 bis 9, bevorzugt 7 bis 8 und besonders bevorzugt 7,2 bis 7,6 haben.

Die erfindungsgemäße Beschichtung ist eine stabile Beschichtung und bietet eine hervorragende Langzeithaltbarkeit. Diese Langzeitstabilität ist bei medizinischen Artikeln eine besonders gewünschte Eigenschaft, da derartige Produkte von medizinischen Einrichtungen im Rahmen rabattierter Großbestellungen erworben werden und bis zu ihrem Einsatz (dessen Zeitpunkt im Regelfall nicht absehbar ist) gelagert werden müssen. Während der Lagerung können die Produkte durchaus saisonal bedingten Temperaturschwankungen unterliegen. Die erfindungsgemäße Beschichtung gewährleistet Beständigkeit sowohl bei langer Lagerungsdauer als auch gegenüber Temperaturschwankungen. Letzteres ist auch einem möglichen Sterilisationsprozess zuträglich.

Die Beschichtung und somit die beschichtete Wundkontaktschicht haben antimikrobielle, insbesondere antibakterielle Eigenschaften, wobei die antibakteriellen Eigenschaften eine Wirkung gegen die humanpathogenen Keime S. *aureus* sowie *P. aeruginosa* umfassen. Die antimikrobielle Wirkung tritt bereits beim Initialkontakt mit den Erregern ein und kann sich im Laufe der Zeit verstärken, wobei eine Kontaktzeit (z.B. Anwendungsdauer auf einer Wunde) von 0 bis 24 Stunden ein bevorzugter Wirkungszeitraum ist.

Bei dem im Rahmen der Erfindung eingesetzten Polypeptid oder Polypeptiden handelt es sich um Polyarginin, Polylysin und / oder Polyornithin. Somit kann jedes Polypeptidmolekül nur Aminosäuren eines einzigen Typs enthalten. Bei Polylysin kann es sich um α-Poly-L-Lysin und / oder ε-Poly-L-Lysin handeln. Das Polylysin kann beispielsweise eine Molekülmasse von 3,5 bis 4 kDa haben. Weiterhin kann das Polylysin pro Molekül 11 bis 40 Untereinheiten, bevorzugt 15 bis 35 Untereinheiten und besonders bevorzugt 20 bis 30 Untereinheiten Lysin umfassen. Ebenso kann das Polyarginin pro Molekül 11 bis 40 Untereinheiten, bevorzugt 15 bis 35 Untereinheiten und besonders bevorzugt 20 bis 30 Untereinheiten Arginin umfassen.

Alle diese genannten Polypeptide tragen aufgrund ihrer chemischen Eigenschaften eine positive Nettoladung und wirken antimikrobiell. Polyarginin hat darüber hinaus zusätzlich den Vorteil die Wundheilung zu fördern, indem der Anteil sogenannter M2 Makrophagen innerhalb der Makrophagen-Population erhöht wird. Während Makrophagen vom Typ M1 Entzündungsreaktionen initiieren und die Produktion von cytotoxischen Radikalen einleiten, wirken M2 Makrophagen entzündungshemmend, proliferativ und fördern den Gewebeverschluss.

Bei dem in der Beschichtung enthaltenen Polypeptid kann es sich auch um Mischungen von Polypeptiden handeln. Insbesondere kann es sich um folgende Mischungen handeln: a) Polyarginin in Kombination mit Polylysin, b) Polyarginin in Kombination mit Polyornithin, c) Polylysin in Kombination mit Polyornithin sowie d) eine Mischung aus allen drei dieser Verbindungen. Das Polypeptid bzw. die Polypeptide sind Teil der erfindungsgemäßen Beschichtung. Dabei kann die Anzahl der Moleküle für die unterschiedlichen Arten von Polypeptiden variabel sein. Auch können in der Beschichtung Polypeptide mit unterschiedlichen Kettenlängen vorhanden sein. Hierrauf wird an anderer Stelle im Detail eingegangen.

Weiterhin können die Polypeptide Mischungen der drei zuvor genannten Aminosäuren in einem Molekül enthalten. Dabei können die Aminosäuren Polyarginin und Polylysin, Polylysin und Polyornithin oder aber Polyarginin und Polyornithin in einem Polypeptid enthalten sein oder das Polypeptid enthält alle drei Aminosäuren.

Der Einsatz polyargininhaltiger Beschichtungen ist selbst bei nicht infizierten Wunden vorteilhaft, da die Heilung beschleunigt wird.

Darüber hinaus bietet die Kombination aus Polyarginin und Polylysin in der Beschichtung eine besonders ausgeprägte antimikrobielle Wirkung, die vermutlich auf einen synergistischen Effekt zurückzuführen ist und die antimikrobielle Wirkung der jeweiligen Einzelsubstanz gegenüber diversen Erregern zu übertreffen vermag.

Bevorzugt liegt die Anzahl der Aminosäuren in einem Polypeptid oder in den Polypeptiden bei mindestens 10. Weiterhin liegt die Anzahl der Aminosäuren in einem Polypeptid oder in den Polypeptiden bevorzugt bei höchstens 2000. Somit haben Polypeptid oder Polypeptide im Rahmen der Erfindung eine bevorzugte Kettenlänge von 10 bis 2000 Aminosäuren, besonders bevorzugt 20 bis 1000 Aminosäuren, ganz besonders bevorzugt 25 bis 100 Aminosäuren und am besten 30 bis 50 Aminosäuren. Wie aus den Ausführungsbeispielen hervorgeht, können auch sämtliche Polypeptide in der Beschichtung jeweils 30 Aminosäuren enthalten oder aus 30 Aminosäuren bestehen. Der daraus resultierende Vorteil ist eine vereinfachte Bereitstellung bei gleichzeitig stark ausgeprägter antimikrobieller Wirkung.

Alternativ kann ein Polypeptid oder Polypeptide mit im Wesentlichen einer einzigen Kettenlänge oder ausschließlich einer einzigen Kettenlänge vorhanden sein. Eine im Wesentlichen einzige Kettenlänge liegt dann vor, wenn mindestens 90 %, besser mindestens 95 %, am besten mindestens 98 % und am allerbesten mindestens 99 % aller in der Beschichtung enthaltenen Polypeptide dieselbe Kettenlänge haben. Eine Beschichtung enthaltend Polypeptide derselben oder im Wesentlichen derselben Kettenlänge bietet den Vorteil, dass die antimikrobielle Wirkung und die Stabilität der Beschichtung sich sehr gut vorhersagen lassen.

Typischerweise sind diese Aminosäuren über Peptidbindungen miteinander verbunden. Die genannten Werte können sich auf einen Teil der Polypeptide in der Beschichtung (z.B. mindestens 90 Gew.-%) oder auf sämtliche Polypeptide in der Beschichtung beziehen.

Hyaluronsäure, auch bekannt als Hyaluronan, ist ein zu den Glycosaminoglycanen zählendes Heteropolysaccharid. Grundbaustein der Hyaluronsäure ist ein aus d-Glucuronsäure und N-Acetyl-d-glucosamin alternierend in (1→3)-(1→4)-β-glycosidischer Bindung aufgebautes Aminodisaccharid. Hyaluronsäure ist Teil der erfindungsgemäßen Beschichtung und trägt aufgrund ihrer chemischen Eigenschaften eine negative Nettoladung. Als negativ geladenes Polymer gehört Hyaluronsäure zu den Polyanionen. Hyaluronsäure ist wasserbindend und hat gewebsregenerierende und wundheilende Eigenschaften. Eine Möglichkeit Hyaluronsäure zu erhalten, ist diese zu synthetisieren, indem Proteine einer bakteriellen Fermentierung unterzogen werden. Durch eine anschließende Filtrierung wird reine Hyaluronsäure gewonnen. Die im Rahmen der vorliegenden Erfindung zu nutzende Hyaluronsäure ist hydrophil und somit löslich in Wasser und den meisten anderen polaren Substanzen.

Hyaluronsäure kann im Rahmen dieser Erfindung in Molmassen von ca. 50 bis ca. 10⁴ kg / mol verwendet werden, bevorzugt wird Hyaluronsäure mit einer molaren Masse von 140 bis 150 kg / mol verwendet. Im Rahmen dieser Erfindung ist es auch möglich, eine Mischung von Hyaluronsäuremolekülen unterschiedlicher Molmassen zu verwenden, wobei in diesem Fall die Molmasse als durchschnittliche Molmasse (Durchschnittsmolmasse) aller Hyaluronsäuremoleküle in der Mischung angegeben werden kann. Beispielsweise kann die durchschnittliche Molmasse 143 bis 146 kg / mol betragen. Alternativ haben alle oder im Wesentlichen alle Hyaluronsäuremoleküle in der Beschichtung dieselbe Molmasse. Eine im Wesentlichen selbe Molmasse liegt vor, wenn mindestens 90 %, besser mindestens 95 %, noch besser mindestens 98 % und am besten mindestens 99 % der Hyaluronsäuremoleküle in der Beschichtung dieselbe Molmasse haben. Generell gilt, dass geeignete Messmethoden zur Molmassenbestimmung wie z.B. die Massenspektrometrie aus dem Stand der Technik bekannt sind.

Die Hyaluronsäure kann quervernetzt oder unvernetzt vorliegen, wobei unvernetzte Hyaluronsäure bevorzugt ist. Gleichzeitig kann auch die antimikrobielle Beschichtung quervernetzt oder unvernetzt vorliegen, wobei unvernetzte Beschichtungen bevorzugt sind. Mögliche Verfahren zur Quervernetzung von Hyaluronsäure sind der Einsatz von 1,4-Butanedioldiglycidylether (BDDE), enzymatische Quervernetzung (z.B. mittels Transglutaminasen) oder physikalische Quervernetzung (z.B. mittels Gefrieren, Erhitzen, Ultraschall, Mikrowellen). Ein mögliches Verfahren zur Quervernetzung von Hyaluronsäure wird in der WO2010131175A1 beschrieben.

Gemäß einem weiteren Aspekt der Erfindung liegt die Hyaluronsäure in der Beschichtung als Polymer vor, wobei es sich um eine Polymermischung mit unterschiedlicher Kettenlänge handeln kann. Dabei kann zumindest ein Teil dieser Polymere eine Molekülmasse von mindestens 10 kDa haben. Bevorzugt hat zumindest ein Teil dieser Polymere der Hyaluronsäure eine Molekülmasse von mindestens 15 kDa, besonders bevorzugt von mindestens 20 kDa, ganz besonders bevorzugt von mindestens 25 kDa und am besten von mindestens 30 kDa. Dieser Teil kann z.B. mindestens 90 Gew.-% der Hyaluronsäure in der Beschichtung ausmachen, bevorzugt mindestens 95 Gew.-% der Hyaluronsäure, besonders bevorzugt mindestens 99 Gew.-% der Hyaluronsäure oder die angegebenen Werte der Molekülmasse können sich alternativ auf die gesamte Hyaluronsäure in der Beschichtung beziehen.

Weiterhin kann die Hyaluronsäure als Polymermischung mit unterschiedlicher Kettenlänge vorliegen, wobei zumindest ein Teil der Hyaluronsäurepolymere in der Polymermischung eine Molekülmasse von mindestens 10 kDa und / oder höchstens 300 kDa hat. Bevorzugt hat zumindest ein Teil der Hyaluronsäurepolymere eine Molekülmasse von höchstens 250 kDa, besonders bevorzugt höchstens 200 kDa, ganz besonders bevorzugt höchstens 150 kDa und am besten höchstens 100 kDa. Dieser Teil kann z.B. 90 Gew.-% der Hyaluronsäure in der Beschichtung ausmachen oder die angegebenen Werte der Molekülmasse können sich alternativ auf die gesamte Hyaluronsäure in der Beschichtung beziehen.

Die erfindungsgemäße antimikrobielle Beschichtung kann mindestens zwei übereinanderliegende und miteinander verbundenen Lagen enthalten, wobei mindestens eine Lage vorhanden ist, welche das Polypeptid oder die Polypeptide enthält und eine positive Nettoladung hat sowie mindestens eine Lage, welche die Hyaluronsäure enthält und eine negative Nettoladung hat, und wobei sich bei den übereinanderliegenden Lagen jeweils eine das Polypeptid oder die Polypeptide enthaltende Lage mit einer die Hyaluronsäure enthaltenden Lage abwechselt, so dass eine Abfolge von alternierenden Lagen gebildet wird.

Die Anzahl der alternierenden Lagen kann dabei eine gerade oder ungerade Zahl sein. Eine gerade Anzahl an alternierenden Lagen ist dabei bevorzugt, weil dabei für jede negativ geladene Lage eine positiv geladene Lage zur Verfügung steht und sich die gegensätzlichen Ladungen anziehen, was zu einer besonders stabilen Beschichtung führt.

Vorzugsweise ist die Anzahl der alternierenden Lagen 20 bis 100, bevorzugt 30 bis 90, besonders bevorzugt 40 bis 80 und ganz besonders bevorzugt 50 bis 70. Dabei enthält jeweils die Hälfte der Lagen die Hyaluronsäure und die andere Hälfte der Lagen das Polypeptid oder die Polypeptide. Bei einer ungeraden Anzahl an alternierenden Lagen überwiegt bevorzugt die Anzahl derjenigen Lagen, die das Polypeptid enthalten.

Wie bereits erläutert kann die Beschichtung der Wundkontaktschicht einen Aufbau aus zwei oder mehr Lagen aufweisen. Der Begriff Lage bezieht sich auf eine Schicht innerhalb der Beschichtung. Eine Lage kann in einem Beschichtungsschritt aufgebracht werden. Die erste Lage wird auf eine Silikonschicht der Wundkontaktschicht aufgebracht. Jede weitere Lage - angefangen mit der zweiten Lage - wird auf die zuletzt aufgebrachte Lage aufgebracht. Dabei kann eine Lage entweder die Hyaluronsäure enthalten und hat somit eine negative Nettoladung oder kann das Polypeptid bzw. die Polypeptide enthaltend und damit eine positive Nettoladung haben.

Darüber hinaus kann die Anzahl der übereinanderliegenden und miteinander verbundenen Lagen in der Beschichtung beispielsweise 10 bis 100 betragen. Bevorzugt beträgt die Anzahl der übereinanderliegenden und miteinander verbundenen Lagen 15 bis 90, besonders bevorzugt 20 bis 80, ganz besonders bevorzugt 25 bis 70 und am besten 30 bis 60.

Eine Möglichkeit die Lagen der Beschichtung aufzutragen, besteht darin, diese mittels Tauchverfahren aufzubringen. Das Tauchverfahren ist darüber hinaus das geeignete Verfahren, um solche Lagen innerhalb der Beschichtung zu erzeugen, die jeweils entweder Hyaluronsäure ohne das Polypeptid bzw. die Polypeptide oder das Polypeptid bzw. die Polypeptide ohne die Hyaluronsäure enthalten. So ist es beim Tauchverfahren möglich zwei Lösungen in zwei separaten Kompartimenten bereitzustellen, wobei ein Kompartiment die Lösung mit der Hyaluronsäure und das andere Kompartiment die Lösung mit dem Polypeptid bzw. den Polypeptiden enthält. Auf die Wundkontaktschicht können auf diese Weise durch abwechselndes Eintauchen in den beiden Kompartimenten Lagen aufgetragen werden, bis die Beschichtung vollständig ist.

Im Rahmen der Erfindung können die Hyaluronsäure und das Polypeptid bzw. die Polypeptide innerhalb der Beschichtung oder innerhalb einer Lage der Beschichtung jedoch auch durchmischt vorliegen. Durchmischt ist in diesem Sinne als Gemisch im chemischen Sinne zu verstehen. Das Gemisch ist zumindest bei dem Beschichtungsprozess bevorzugt eine Lösung. Bevorzugt sind die Hyaluronsäure und das Polypeptid bzw. die Polypeptide innerhalb der Beschichtung homogen verteilt. Weiterhin bevorzugt ist das Polypeptid oder sind die Polypeptide in eine Hyaluronsäurematrix eingebettet. Eine solche Matrix kann als Hyaluronsäuregel vorliegen und Hyaluronsäure sowie Wasser enthalten. Die Hyaluronsäurematrix bildet sich aus, wenn die Beschichtung im trockenen Zustand vorliegt oder durch Trocknung in diesen übergeht, wobei eine Restfeuchtigkeit durch chemische Wechselwirkungen erhalten bleibt.

Eine durchmischte Beschichtung im Sinne des eben Genannten lässt sich durch Aufsprühen einer Lösung enthaltend Hyaluronsäure und Aufsprühen einer Lösung enthaltend das Polypeptid bzw. die Polypeptide auf die Wundkontaktschicht erhalten. Die beiden Lösungen können nacheinander oder gleichzeitig gesprüht werden. Mögliche Verfahren sind in den Ausführungsbeispielen näher erläutert. Sobald eine Lage erhalten wurde und diese getrocknet oder angetrocknet ist, können weitere Lagen aufgesprüht oder mittels Tauchverfahren hinzugefügt werden.

Eine durchmischte Beschichtung kann eine oder mehrere Lagen enthalten, von denen mindestens eine Lage sowohl die Hyaluronsäure als auch das Polypeptid bzw. die Polypeptide enthält. In diesem Sinne ist eine solche durchmischte Beschichtung gänzlich oder möglicherweise nur teilweise von der Mischung durchdrungen. Die Beschichtung kann eine oder mehrere durchmischte Lagen enthalten.

Die Wundkontaktschicht kann die antimikrobielle Beschichtung in folgenden Varianten enthalten, wobei diese Aufzählung exemplarisch und nicht abschließend zu verstehen ist:
a) Beschichtung umfassend mindestens zwei Lagen, von denen mindestens eine Lage Hyaluronsäure, aber kein Polypeptid enthält und von denen mindestens eine Lage ein Polypeptid oder Polypeptide aber keine Hyaluronsäure enthält. Falls mehr als zwei Lagen vorhanden sind, sind diese alternierend angeordnet.
b) Durchmischte Beschichtung umfassend mindestens eine Lage, die sowohl Hyaluronsäure als auch das Polypeptid bzw. die Polypeptide enthält.
c) Eine Kombination aus a) und b).

Falls nur die wundzugewandte (proximale) Seite der Wundkontaktschicht beschichtet ist, können die distale oder proximale Seite der Wundkontaktschicht durch unterschiedliche Farbgebung als Ober- oder Unterseite für den Nutzer gekennzeichnet sein. Auf diese Weise ist für den Nutzer erkennbar, welche Seite der Wundkontaktschicht auf die Wunde aufzulegen ist.

Gemäß einem Konzept der Erfindung haben das Polypeptid bzw. die Polypeptide der antimikrobiellen Beschichtung eine Molekülmasse von 1 bis 41 kDa. Bevorzugt haben das Polypeptid bzw. die Polypeptide eine Molekülmasse von 2 bis 40 kDa, besonders bevorzugt 3 bis 39 kDa, ganz besonders bevorzugt 4 bis 38 kDa und am besten 5 bis 37 kDa.

Die Beschichtung der Wundkontaktschicht kann eine Dicke von 10 nm bis 1000 nm haben. Bevorzugt hat die Beschichtung eine Dicke von 50 nm bis 900 nm, besonders bevorzugt eine Dicke von 100 nm bis 800 nm, besonders bevorzugt eine Dicke von 150 nm bis 700 nm und am besten 200 nm bis 600 nm. Die Dicke kann dabei gemessen werden von der Oberkante der Grundfläche der unbeschichteten Wundkontaktschicht, und zwar ohne, dass die Wundkontaktschicht während der Messung gestaucht oder komprimiert wird. Die Werte beziehen sich auf die Dicke der Beschichtung nach aktiver oder passiver Rücktrocknung.

Es wird empfohlen die Dicke der Beschichtung durch die Anzahl der Lagen einzustellen. Die Dicke nimmt mit zunehmender Anzahl der Lagen weiter zu. Beschichtungen, deren Dicke eine größere Ausdehnung erreicht, haben besonders ausgeprägte atraumatische Eigenschaften. Beschichtungen, die eine geringe Dicke aufweisen, ermöglichen die Einsparung von Beschichtungsmaterial.

Die Wundkontaktschicht kann eine Beschichtung mit einem Flächengewicht von beispielsweise 5 ng bis 200 ng / cm² enthalten. Bevorzugt hat die Beschichtung ein Flächengewicht von 10 ng bis 150 ng / cm², besonders bevorzugt ein Flächengewicht von 15 ng bis 130 ng / cm², ganz besonders bevorzugt ein Flächengewicht von 20 ng bis 100 ng / cm² und am besten 20 ng bis 50 ng / cm². Das Flächengewicht der Beschichtung bezieht sich dabei auf die Summe der Massen aus Polypeptid bzw. Polypeptiden und Hyaluronsäure. Bei der Bestimmung des Flächengewichts können etwaige Perforationen der Wundkontaktschicht unberücksichtigt bleiben. Beispielsweise kann bei einer perforierten Wundkontaktschicht mit einer Fläche von 10 cm x 10 cm davon ausgegangen werden, dass die Fläche der Wundkontaktschicht 100 cm² beträgt.

Ebenso kann das Flächengewicht des Polypeptids bzw. der Polypeptide in der Beschichtung beispielsweise 1 ng bis 180 ng / cm², bevorzugt 2 ng bis 100 ng / cm² und ganz besonders bevorzugt 3 ng bis 50 ng / cm² Wundkontaktschicht betragen.

Ebenso kann das Flächengewicht der Hyaluronsäure in der Beschichtung beispielsweise 1 ng bis 180 ng / cm², bevorzugt 2 ng bis 100 ng / cm² und besonders bevorzugt 3 ng bis 50 ng / cm² Wundkontaktschicht betragen.

Das Verhältnis von Polypeptid bzw. Polypeptiden zu Hyaluronsäure in der Beschichtung kann beispielsweise ein Massenverhältnis von 0,5 zu 1 bis 5 zu 1 haben. Bevorzugt liegt das Verhältnis bei 1 zu 1 bis 4 zu 1, besonders bevorzugt liegt das Verhältnis bei 1,5 zu 1 bis 3,5 zu 1 und ganz besonders bevorzugt bei 2 zu 1 bis 3,5 zu 1.

Des Weiteren können die Inhaltsstoffe der antimikrobiellen Beschichtung in einem bestimmten Mengenverhältnis zueinander stehen. So kann das Gewichtsverhältnis der Gewichtskonzentration der Hyaluronsäure zur Gewichtskonzentration des Polypeptids bzw. der Polypeptide in der Beschichtung von 9 zu 1 bis hin zu 1 zu 80 reichen, bevorzugt 4 zu 1 bis hin zu 1 zu 20.

Ferner kann das Stoffmengenverhältnis der Stoffmenge der Hyaluronsäure zur Stoffmenge des Polypeptids bzw. der Polypeptide von 4 zu 25 bis hin zu 1 zu 500 reichen. Die Stoffmengenbestimmung kann in der Standardeinheit mol vorgenommen werden.

Die Wundkontaktschicht ist teilweise oder vollständig mit der Beschichtung beschichtet. Insbesondere können mindestens 80 % der Fläche der wundzugewandten (proximalen) Seite beschichtet sein. Bevorzugt sind 90 % der Fläche der wundzugewandten Seite beschichtet. Besonders empfohlen wird mindestens 99 % dieser Fläche zu beschichten. Perforationen können bei der Flächenbestimmung unberücksichtigt bleiben.

Die applizierte Beschichtungsmenge kann durch Wiegen der beschichteten Wundkontaktschicht ermittelt werden. Beschichtungen mit größerem Flächengewicht können durch wiederholte Einzelbeschichtungen erzeugt werden.

Weiterhin ist die erfindungsgemäße Beschichtung vorzugsweise trocknungsbeständig. In diesem Sinne können die Bestandteile der Beschichtung - wie Hyaluronsäure und Polypeptid oder Polypeptide - im feuchtem Zustand als Lösung auf die Wundkontaktschicht aufgetragen werden, um anschließend zu trocknen oder getrocknet zu werden. Das Trocknen kann passiv bei Raumtemperatur erfolgen oder aktiv unter Einsatz technischer Hilfsmittel, wobei letzteres im Allgemeinen deutlich schneller, aber auch energieaufwändiger ist. Die Beschichtung behält auch im getrockneten Zustand ihre Eigenschaften - insbesondere die atraumatische und antimikrobielle Wirkung - bei. Die Trocknungsbeständigkeit der Beschichtung hat den Vorteil, dass sich trockene Wundversorgungsprodukte einfacher im industriellen Maßstab verarbeiten und verpacken lassen. Darüber hinaus lassen sich trocknungsbeständige Wundversorgungsprodukte leichter und länger lagern, da sie nicht gegen ein ungewolltes Eintrocknen geschützt werden müssen.

Eine Restfeuchtigkeit kann auch in der getrockneten bzw. trockenen Beschichtung aufgrund der hygroskopischen Eigenschaften der Inhaltsstoffe vorhanden sein. Die Beschichtung gilt beispielsweise als trocken, wenn der Flüssigkeitsgehalt (z.B. Wassergehalt) in der Beschichtung maximal 5 Gew.-%, bevorzugt maximal 4 Gew.-%, besonders bevorzugt maximal 3 Gew.-%, ganz besonders bevorzugt maximal 1 Gew.-% und am besten maximal 0,1 Gew.-% beträgt.

Die Öffnungen der Wundkontaktschicht können eine Fläche oder Durchschnittsfläche von 0,1 mm² bis 50 mm² pro Öffnung haben, bevorzugt 1 mm² bis 15 mm² und besonders bevorzugt 2 mm² bis 5 mm². Bei kreisförmigen oder im wesentlichen kreisförmigen Öffnungen kann der durchschnittliche Durchmesser 0,1 mm bis 30 mm, vorzugsweise 0,5 mm bis 3 mm betragen. Öffnungen, die auf diese Weise beschaffen sind, erlauben den Durchtritt von Flüssigkeiten und vermitteln der Wundkontaktschicht gleichzeitig starke atraumatische Eigenschaften. Die Öffnungen können in ihrer Größe variieren.

Die erfindungsgemäße Wundkontaktschicht enthält Silikon. Bei dem Silikon kann es sich um ein Silikongel handeln. Das Silikongel kann adhäsive Eigenschaften haben, wobei auch bei adhäsivem Silikon die atraumatischen Eigenschaften erhalten bleiben. Überraschenderweise hat sich gezeigt, dass die adhäsiven Eigenschaften des Silikongels auch nach Auftragung der antimikrobiellen Beschichtung erhalten bleiben, so dass es nun möglich ist, antimikrobielle, atraumatische und gleichzeitig adhäsive Wundkontaktschichten zu erhalten. Im Rahmen der Erfindung kann es sich bei dem Silikon der silikonhaltigen Wundkontaktschicht um ein Silikongel handeln, welches zur atraumatischen Befestigung der Wundkontaktschicht an menschlicher Haut geeignet ist. Bei diesem Silikongel kann es sich um ein Polydimethylsiloxan-basiertes Silikongel handeln. Das Silikongel kann auf eines der hierin beschriebenen Substrate aufgetragen sein. In diesem Fall ist zumindest die zur Wunde ausgerichtete Seite des Substrates mit Silikongel ausgestattet.

Geeignetes Silikon für den Einsatz in der Wundkontaktschicht kann erhalten werden durch Polykondensation einer Zusammensetzung umfassend Dimethyldichlorsilan und/oder Trimethylmonochlorsilan sowie Einheiten aus der Gruppe bestehend aus (i) Monomethyltrichlorsilan, und/oder (ii) einem Element der Gruppe bestehend aus Maleinsäure, Fumarsäure und Trans-3-Hexendisäure sowie Mischungen davon.

Weiterhin kann geeignetes Silikon ausgewählt sein aus der Gruppe bestehend aus Vinyl-Methyl-Silicon, Phenyl-Vinyl-Methyl-Silicon und Fluor-Vinyl-Methyl-Silicon. Diese Silikonarten können zur weiteren Quervernetzung einer ionisierenden Bestrahlung ausgesetzt werden. Dabei kann eine Energiedosis von 5-60 kGy appliziert werden. Höhere Energiedosen führen zu einer stärkeren Quervernetzung und zu einer Abnahme der Adhäsivität des Silikons. Ein adhäsives Silikongel kann durch Weglassen von Quervernetzungsmaßnahmen oder durch Applikation einer ionisierenden Bestrahlung mit niedriger Energiedosis erhalten werden. Während der Einstellung der Quervernetzung mittels ionisierender Strahlung kann das Silikon gleichzeitig durch die Strahlung sterilisiert werden, was eine Ersparnis von Zeit und Energie erlaubt.

Ferner ist es möglich für die Wundkontaktschicht ein Silikon mit absorbierenden und desorbierenden Eigenschaften zu verwenden. Ein solches Silikon verbessert die Atmungsaktivität der Wundkontaktschicht und bei einem Einsatz von Perforationen kann eine kleinere Gesamtperforationsfläche gewählt werden, wodurch die adhäsiv wirksame Fläche der Wundkontaktschicht zunehmen kann, was eine verbesserte Anhaftung ermöglicht. Das Silikon mit absorbierenden und desorbierenden Eigenschaften kann Partikel eines Alkalisalzes eines Polymers enthalten, das Carboxylatgruppen enthält, wobei die Teilchen einen durchschnittlichen Teilchendurchmesser D50 von 1 bis 40 µm aufweisen. Bevorzugt haben die Partikel einen durchschnittlichen Teilchendurchmesser D50 von 4 bis 30 µm. Bei dem Polymer kann es sich um ein Poly(meth)acrylat handeln. Die Partikel können 5 to 50 Gew.-% des Silikons oder der silikonhaltigen Wundkontaktschicht ausmachen und eine Porosität von 10 bis 75% aufweisen. Eine silikonhaltige Wundkontaktschicht, welche die erwähnten Partikel enthält, kann eine Durchlässigkeitsrate für Wasserdampf (MVTR) von 1500 to 25000 g/m²/24h haben, wodurch sich das Ansammeln von Schweiß unterhalb der Wundkontaktschicht reduzieren oder gänzlich verhindern lässt. Dies verbessert das Empfinden für Patienten und verhindert eine Abnahme der Adhäsionskraft. Das partikelhaltige Silikon der Wundkontaktschicht kann im Wesentlichen oder vollständig frei sein von Polyacrylat, MQ Harz und / oder pyrogener Kieselsäure.

Das Silikon der Wundkontaktschicht kann als flexibler und elastischer Festkörper vorliegen, der sich der Form der Körper- oder Wundoberfläche anpassen kann. Alternativ kann das Silikon auch nur einen Teil eines solchen Festkörpers ausmachen und der Festkörper enthält neben dem Silikon ein oder mehrere andere Materialien.

Vorzugsweise enthält zumindest die proximale Seite der Wundkontaktschicht Silikon oder aber zumindest die proximale Seite der Wundkontaktschicht ist mit Silikon überzogen. Die Wundkontaktschicht ein Substrat enthalten. Das Substrat kann mit Silikon überzogen sein. Das Substrat kann auch allseitig mit Silikon überzogen sein. Ein solches Substrat besteht aus einem unlöslichen und medizinisch akzeptables Material. Das Material kann über eine Kristallgitterstruktur verfügen oder alternativ als teilkristalliner oder amorpher Festkörper vorliegen (z.B. als amorpher Thermoplast wie Polyvinylchlorid). Bei dem Material kann es sich um ein Polymer- oder um eine Polymermischung handeln, insbesondere aus einem thermoplastischen Polymer oder einer Mischung thermoplastischer Polymere. Beispielsweise kann das Substrat Polyethylenterephthalat (PET) enthalten oder daraus bestehen.

Sofern die Wundkontaktschicht ein Substrat enthält, kann das Substrat einen Kunststoff oder ein Kunststoffgemisch enthalten. Somit kann es sich bei dem Substrat um ein Polymersubstrat handeln. Der Kunststoff kann ein reiner Kunststoff sein oder ein Gemisch aus zwei oder mehr Kunststoffen. Beispiele für geeignete Kunststoffe sind Polyethylen, Polypropylen, Polyester, Polyethylenterephthalat, Polyamid, Polyvinylchlorid, Polyacrylat, Polymethylmethacrylat sowie Polyurethan. Das Substrat kann beispielsweise mindestens 70-Gew.-%, mindestens 80-Gew.-%, mindestens 90 Gew.-% oder mindestens 99 Gew.-% Polyethylenterephthalat oder Polyurethan enthalten. Weiterhin kann das Substrat vollständig aus Polyethylen oder Polyurethan bestehen. Polyethylenterephthalat hat den Vorteil, dass es elastisch und biegsam ausgestaltet werden kann, sich leicht verarbeiten lässt und nicht mit körpereigenen Stoffen reagiert.

Das Substrat kann insbesondere als Folie vorliegen. Die Folie kann eine Dicke von 5 µm bis 50 µm und bevorzugt 10 µm bis 40 µm haben. Derartige Dicken eignen sich besonders gut zur Ausbildung von Laminaten Ein bevorzugter Kunststoff für die Ausbildung einer Folie ist Polyurethan. Polyurethan ist zäh und eignet sich für die Ausbildung sehr dünner und dennoch reißfester Folien.

Sofern ein Substrat vorhanden ist, ist bevorzugt zumindest die gesamte proximale Fläche des Substrats wundseitig von Silikon bedeckt bzw. beschichtet. Perforationen sind dennoch möglich. Es hat sich gezeigt, dass es vorteilhaft ist, wenn Substrat und Silikon im Wesentlichen dieselbe Ausdehnung (Fläche) haben und kongruent (deckungsgleich) zueinander sind, so dass keine der beiden die jeweils andere nennenswert überragt. Auf diese Weise ergänzen sich Substrat und Silikon optimal in ihrer Funktion.

Ferner kann das Substrat allseitig von Silikon überzogen sein. Diese Überzug kann eine Dicke von 20 µm bis 225 µm und bevorzugt 40 µm bis 200 µm haben. Bei einem allseitigen Überzug besteht die angegebene Dicke sowohl distal als auch proximal des Substrates. Die Dicke kann dabei ausgehend vom Substrat gemessen werden.

Das Silikon der Wundkontaktschicht kann daneben auch als Schicht vorliegen. Dabei ist es möglich, dass die Wundkontaktschicht über genau eine Silikonschicht verfügt. Die Schicht kann Teil eines Laminats sein. In einem solchen Fall ist die silikonhaltige Wundkontaktschicht als Laminat ausgestaltet. Das Laminat enthält ein Substrat.

Bei dem Substrat kann es sich um eine Folie handeln. Die Folie kann sich an der wundabgewandten Seite der Wundkontaktschicht und somit oberhalb (distal) zur Silikonschicht befinden. Die Silikonschicht kann eine Dicke von 20 µm bis 225 µm und bevorzugt 40 µm bis 200 µm haben. Die Silikonschicht kann wundseitig mit der antimikrobiellen Beschichtung beschichtet sein, so dass die antimikrobielle Beschichtung an der Silikonschicht haftet.

Die distale Seite eines solchen Laminats - also die wundabgewandte Seite eines Substrats wie einer Folie - kann eine Klebebeschichtung aufweisen. Hierzu kann das Substrat teilweise oder vollständig mit einem Adhäsiv behandelt sein. Bei dem Adhäsiv kann es sich um ein Adhäsiv auf Acrylbasis, um ein Hydrokolloid, einen Klebstoff auf Harzbasis oder einen Klebstoff auf Basis von Polyurethan oder Polyurethan-Copolymer handeln. Die mit dem Adhäsiv behandelte Seite des Laminats kann verwendet werden, um weitere Schichten (z.B. eine Absorptionsschicht) anzubringen und bis dahin von einer Schutzschicht bedeckt sein, die erst unmittelbar vor Einbau weiterer Schichten entfernt wird.

Eine bevorzugte Art einer Wundkontaktschicht in Form eines Laminats ist eine Folie aus Polyurethan, welche wundseitig eine Schicht aus Silikongel trägt und an der gegenüberliegenden Seite (distal) einen Auftrag eines Adhäsivs auf Acrylbasis. Das Silikongel ist mit der erfindungsgemäßen antimikrobiellen Beschichtung ausgestattet. Das Laminat ist perforiert. Die distale Seite der Polyurethanfolie kann bis zur Benutzung von einer Schutzschicht bedeckt sein, die das Adhäsiv bedeckt.

Bevorzugt ist die Wundkontaktschicht selbst faserlos ausgestaltet. Durch die Abwesenheit von Fasern hat die Wundkontaktschicht selbst weder eine Dochtwirkung noch absorbierende Eigenschaften und deswegen kaum Interaktion mit Wundflüssigkeiten. Hierdurch erhält die Wundkontaktschicht ausgeprägt atraumatische Eigenschaften, die durch die ebenfalls atraumatisch wirkende antimikrobielle Beschichtung weiter verstärkt werden. Wenn die Wundkontaktschicht Teil einer Wundauflage ist, kann die Wundauflage Fasern enthalten, welche bevorzugt ausschließlich distal zur Wundkontaktschicht liegen.

Die erfindungsgemäße Wundkontaktschicht kann Teil einer absorbierenden Wundauflage sein, welche eine Absorptionsschicht enthält. Eine solche absorbierende Wundauflage ist ebenfalls von der Erfindung umfasst.

Überraschenderweise hat sich gezeigt, dass die Beschichtung zwar ein stabiler Teil der Wundkontaktschichtf ist, aber dennoch durchleitend für Blut und wässrige Wundflüssigkeiten wie Wundexsudat ist. Das heißt, dass Blut und Wundexsudat die Beschichtung passieren können. Flüssigkeiten können an weitere optional vorhandene Schichten, die sich distal zur Wundkontaktschicht befinden, weitergegeben werden. Bevorzugt handelt es sich dabei um eine oder mehrere Absorptionsschichten. Mithilfe absorbierender Materialien kann dabei ein Sogeffekt und / oder Dochteffekt generiert werden, der Flüssigkeit von der wundzugewandten Seite zur distalen Seite der Wundkontaktschicht leitet. Die Durchflussrate kann dabei anhand der Anzahl der Perforationen in der Wundkontaktschicht, der Gesamtfläche der Perforationen in der Wundkontaktschicht und optional in der Art und Menge der verwendeten Absorptionsmittel eingestellt werden. Die Erfindung umfasst in diesem Sinne auch eine perforierte Variante der erfindungsgemäßen Wundkontaktschicht, die durchlässig für Flüssigkeiten wie Wundexsudat oder Blut ist.

Eine distal zur Wundkontaktschicht angeordnete Absorptionsschicht kann die folgenden Materialien oder Materialgemische enthalten oder daraus bestehen: Kunststofffasern, Polyolefin basierte Fasern, Polyethylen, Polyetheretherketon (PEEK), Polyvinylchlorid (PVC), Polymethylmethacrylat (PMMA), Polykarbonat (PC), Polyester, Polyethylenterephthalat (PET), Polypropylen (PP), Polystyren (PS), Polyamid, Acrylnitril-Butadien-Styrol (ABS), Polylactide (PLA), Viskose, Zellulose basierte Fasern oder Mischungen aus zwei oder mehr der zuvor genannten Materialien. Bei Verwendung von Polyamid als Fasermaterial kann das Polyamid in Form von Nylon vorliegen. Mögliche Mischungen sind eine Mischung aus Polypropylen und Viskose, eine Mischung aus Polyethylen und Viskose, eine Mischung von Polypropylen, Polyethylen und Viskose, eine Mischung aus Polyester mit Baumwolle oder eine Mischung aus Polyester mit Viskose. Darüber hinaus können auch Mischfasern verwendet werden, welche eine Kombination aus zwei oder mehr der aufgeführten Materialien in einer Faser vereinen. Bei der Absorptionsschicht kann es sich um ein Vliesmaterial handeln, welches die oben genannten Faserarten enthält.

Synthetische Fasern wie beispielsweise Polypropylen und Polyethylen haben den Vorteil, dass sie beständig gegen Zersetzung durch Mikroorganismen sind. Weiterhin haben viele dieser Fasern wie beispielsweise Polypropylen hervorragende thermoplastische Eigenschaften, was das Verarbeiten und Zusammenfügen von Lagen (z.B. mittels Schweißen) vereinfacht. Materialien, die dieselben chemischen Verbindungen enthalten, bilden beim Schweißen besonders stabile Nähte aus.

Möglich ist, dass die Absorptionsschicht ausschließlich solche Fasern enthält, die synthetischen Ursprungs sind und / oder solche Fasern, die zu den Kunstfasern gezählt werden. Viskose ist dabei eine synthetische Faser, die im Rahmen der vorliegenden Erfindung jedoch nicht zu den Kunstfasern gezählt wird.

Weiterhin ist auch möglich, dass die Absorptionsschicht ausschließlich solche Fasern enthält, die natürlichen Ursprungs sind und / oder biologisch abbaubar sind. Bei der biologischen Abbaubarkeit kann es sich um eine Abbaubarkeit im Sinne der Norm EN ISO 14851 :2019 handeln. Fasern natürlichen Ursprungs sind beispielsweise Baumwollfasern. Fasern, die im Kontext der vorliegenden Erfindung als biologisch abbaubar gelten, sind beispielsweise Baumwollfasern und Viskosefasern. Im Gegensatz dazu sind Kunstfasern nicht biologisch abbaubar.

Bevorzugt enthält die Absorptionsschicht Fasern aus Polypropylen und / oder Polyethylen. Der Anteil von Polypropylen und / oder Polyethylen kann 2 Gew.-% bis 40 Gew.-% betragen. Bevorzugt beträgt der Anteil 5 Gew.-% bis 30 Gew.-%, besonders bevorzugt 7 Gew.-% bis 20 Gew.-% und ganz besonders bevorzugt 9 Gew.-% bis 16 Gew.-%. Polypropylen hat vorteilhafte thermoplastische Eigenschaften. Darüber hinaus ist es äußerst reißfest. Polyethylen hat den Vorteil elastisch zu sein und ist darüber hinaus geeignet als Verstärkungsfaser zu dienen. Weiterhin kann die Absorptionsschicht eine Mischung aus Fasern von Viskose, Polyethylen sowie Polypropylen und / oder Polyethylenterephthalat enthalten. Bevorzugt enthält das absorbierende Vliesmaterial Fasern aus Viskose, Polyethylen und entweder
a) Polypropylen oder
b) Polyethylenterephthalat,
wobei optional mindestens 80 Gew.-% des absorbierenden Vliesmaterials aus Viskose bestehen können. Bevorzugt bestehen mindestens 84 Gew.-% des absorbierenden Vliesmaterials aus Viskose, besonders bevorzugt mindestens 89 Gew.-%. Die Viskose hat als hydrophile Faser eine hervorragende Dochtwirkung und verleiht dem Vliesmaterial absorbierende Eigenschaften. Unabhängig davon kann der Anteil von der Summe von Polyethylen und Polypropylen in der Absorptionsschicht mindestens 7 Gew.-%, bevorzugt mindestens 8 Gew.-%, besonders bevorzugt mindestens 9 Gew.-% und am besten mindestens 10 Gew.-% betragen. Im Falle eines Anteils von mindestens 10 Gew.-% könnte das Vliesmaterial beispielsweise 5 Gew.-% Polyethylen und 5 Gew.-% Polypropylen enthalten und der übrige Anteil könnte Viskose sein.

Die Wundkontaktschicht kann auf unterschiedliche Weise mit einer Absorptionsschicht verbunden werden. So können Wundkontaktschicht und Absorptionsschicht unter Einsatz eines Klebemittels (z.B. mittels eines Adhäsives auf Acrylbasis) aneinander befestigt werden. Eine andere Möglichkeit ist die thermische Verfestigung - beispielsweise mittels Kalanderverfestigung oder Heißluftverfestigung - oder das Schweißen. Optional kann sich zwischen der Wundkontaktschicht und der Absorptionsschicht ein faserloses Kunststoffnetz befinden. Bevorzugt besteht ein solches faserloses Kunststoffnetz aus Polyethylen.

Daneben ist der Einbau saugfähiger Materialien, welche das hindurchtretende Exsudat in der Wundauflage speichern möglich. Diese können oberhalb (distal) von der Absorptionsschicht als zusätzliche Retentionsschicht vorliegen oder Teil der Absorptionsschicht selbst sein. Beispiele für geeignete Materialien zur Speicherung von Exsudat sind Superabsorber und Zellstoffflocken. Die Zellstofflocken können vorwiegend oder vollständig aus Zellulose bestehen. Bei dem Superabsorber kann es sich um ein superabsorbierendes Polymer handeln, welches in der Regel auf Basis von Acrylsäure ausgebildet ist. Das superabsorbierende Polymer kann in Form von Partikeln oder superabsorbierenden Fasern vorkommen. Eine mögliche Ausgestaltung ist eine oberhalb (distal) der Absorptionsschicht angebrachte Retentionsschicht, welche superabsorbierende Fasern enthält. Eine andere mögliche Ausgestaltung ist eine Absorptionsschicht in Form einer Tasche, welche mit superabsorbierenden Partikeln und Zellstoffflocken gefüllt ist. Die Tasche selbst kann dabei aus einem Vlies ausgebildet sein.

Bei der Speicherung der aufgenommenen Flüssigkeit reagiert die Flüssigkeit mit dem Superabsorber zu einer gelartigen Quellmasse, welche in der Retentionsschicht eingeschlossen ist. Durch das Aufquellen der superabsorbierenden Substanzen wird die antimikrobielle Beschichtung in Richtung Wundbett gedrückt und der Kontakt zwischen antimikrobieller Beschichtung und Wundbett intensiviert. Dies funktioniert besonders gut, wenn die Wundkontaktschicht Teil einer Wundauflage mit Superabsorber und einem Backing mit umlaufenden Kleberand ist, wobei das Backing als Widerlager fungiert.

Demgemäß kann die erfindungsgemäße Wundauflage ein Backing als distale Abschlussschicht enthalten. Die proximale Seite dieses Backings kann mit einer adhäsiven Beschichtung ausgestattet sein, um das Backing mit dem Rest der Wundauflage zu verbinden. Die Flächenausdehnung kann kongruent sein mit den übrigen Teilen der Wundauflage (z.B. gleiche Flächenausdehnung haben wie die Wundkontaktschicht) oder diese überragen. Wenn das adhäsive Backing die übrigen Bestandteile der Wundauflage allseitig überragt, wird ein umlaufender wundseitiger Kleberand ausgebildet, der während des Gebrauchs eine Anhaftung der Wundauflage - z.B. an die Haut eines Patienten - ermöglicht. Alternativ können Wundkontaktschicht und Backing eine dazwischenliegende Absorptionsschicht und ggf. eine optional vorhandene Retentionsschicht allseitig umgeben, so dass Absorptionsschicht und optional auch Retentionsschicht allseitig von Backing und Wundkontaktschicht umschlossen sind. Das Backing und die Wundkontaktschicht können dabei in einem die Absorptionsschicht und optional auch Retentionsschicht umlaufenden Rand Kontakt zueinander haben und miteinander verbunden sein, so dass eine sogenannte Sandwich-Variante einer Wundauflage ausgebildet wird. Diese Verbindung kann durch Einsatz von einem oder aber zwei Adhäsiven zustande kommen. Zwei Adhäsive kommen zum Einsatz, wenn die Wundkontaktschicht als adhäsiv behandeltes Laminat vorliegt und gleichzeitig auch das Backing mit einem Adhäsiv behandelt ist. Eine mögliche Kombination ist das Acryl-basierte Adhäsiv im Laminat und ein harzbasiertes Adhäsiv auf dem Backing.

Das Backing selbst kann aus einer Folie oder einem Vliesmaterial bestehen. Ein Backing aus einer Folie kann undurchdringlich für flüssiges Wasser sein, aber durchlässig gegenüber Wasserdampf. Im Falle, dass eine Backingfolie eingesetzt wird, kann diese Polyurethan beinhalten oder daraus bestehen. Im Falle, dass ein Vliesmaterial das Backing bildet, kann dieses Vliesmaterial aus den gleichen oder anderen Fasern oder Fasergemischen bestehen wie die Absorptionsschicht. Vorzugsweise besteht ein Vliesbacking aus Polyester.

Die fertige Wundkontaktschicht kann an ihrer beschichteten Wundkontaktfläche durch eine Schutzschicht wie beispielsweise einen Release-Liner überdeckt sein. Eine solche Schutzschicht wird vom Anwender vor der Verwendung entfernt.

Ferner von der Erfindung umfasst sind Verfahren zur Herstellung der hierin beschriebenen Wundkontaktschicht und zum Beschichten von Wundkontaktschichten mit der erfindungsgemäßen antimikrobiellen Beschichtung.

Nachfolgend wird ein Verfahren beschrieben, mit dem man eine silikonhaltige Wundkontaktschicht mit durchmischter Beschichtung und / oder mit mindestens einer durchmischten Lage innerhalb der Beschichtung erzeugen kann:
a) Bereitstellen einer silikonhaltigen Wundkontaktschicht,
b) Besprühen zumindest einer Seite der bereitgestellten Wundkontaktschicht mit i) einer Hyaluronsäure und ii) einem Polypeptid, um eine Beschichtung auszubilden, welche mindestens eine Beschichtungslage umfasst, wobei das Polypeptid ausgewählt ist aus Polyarginin, Polylysin und Polyornithin oder einer Mischung aus mindestens zwei der zuvor genannten Polypeptide und wobei die mindestens eine Beschichtungslage sowohl die Hyaluronsäure als auch das Polypeptid enthält.
c) optionale ein- oder mehrmalige Wiederholung von Schritt b)
d) optionale Rücktrocknung der beschichteten Wundkontaktschicht.

Bevorzugt wird in Schritt b) zumindest die proximale (in Benutzung zur Wunde ausgerichtete) Seite der Wundkontaktschicht beschichtet. Es muss dabei nicht die gesamte Fläche der proximalen Seite der Wundkontaktschicht beschichtet werden. Eine Teilbeschichtung ist ebenfalls möglich.

Das Besprühen kann beispielsweise mittels eines Zerstäubers oder einer Sprühpistole geschehen. Bevorzugt handelt es sich um ein elektrisches Sprühgerät, das eine konstante Menge an Flüssigkeitsvolumen pro Zeiteinheit versprüht.

Einer der großen Vorteile dieses Verfahrens ist die Zeitersparnis. In diesem Sinne kann vorgesehen sein, dass das Aufsprühen einer Lage maximal 5 min, bevorzugt maximal 1 min, besonders bevorzugt maximal 30 s und ganz besonders bevorzugt maximal 5 s dauert.

Weiterhin kann vorgesehen sein, dass der gesamte Beschichtungsprozess mittels Aufsprühen vom Beginn des Sprühvorgangs bis zum Erhalt der fertigen antimikrobiell beschichteten Wundkontaktschicht maximal 30 min dauert, wobei die Trocknung der Wundkontaktschicht in diesem Zeitraum bereits inkludiert sein kann.

Bevorzugt liegen die Hyaluronsäure und das Polypeptid oder die Polypeptide während des Besprühens gemäß Schritt b) des obigen Herstellungsverfahrens in Lösung vor. Mögliche Lösungsmittel wie polare Flüssigkeiten, insbesondere Wasser und wässrige Pufferlösungen werden hierin beschrieben und können im Rahmen von dem Herstellungsverfahren eingesetzt werden.

Bevorzugt beträgt die Konzentration des in Lösung vorliegenden Polypeptids oder der Polypeptide während des Besprühens gemäß Schritt b) 0,1 mg / ml bis 100 mg / ml, besonders bevorzugt 1 mg / ml bis 80 mg / ml, ganz besonders bevorzugt 3 mg / ml bis 50 mg / ml und am besten 5 mg / ml bis 30 mg / ml.

Bevorzugt beträgt die Konzentration der Hyaluronsäure während des Besprühens gemäß Schritt b) bei 0,1 mg / ml bis 10 mg / ml, besonders bevorzugt 0,5 mg / ml bis 8 mg /ml, ganz besonders bevorzugt 1 mg / ml bis 5 mg / ml und am besten 2 mg / ml bis 4 mg / ml.

Bevorzugt werden durch das Besprühen gemäß Schritt b) 0,1 bis 1 ml einer solchen Lösung des Polypeptids bzw. der Polypeptide und / oder 0,1 ml bis 1 ml einer solchen Lösung der Hyaluronsäure pro cm² der proximalen (wundzugewandten) Seite der Wundkontaktschicht aufgesprüht. Besonders bevorzugt werden 0,2 bis 0,9 ml, ganz besonders bevorzugt 0,3 bis 0,8 ml und am besten 0,4 bis 0,7 ml einer solchen Lösung aufgesprüht. Die Volumenangaben sind pro Lage zu verstehen. Da im Falle durchmischter Lagen bereits eine einzige Lage eine fertige Beschichtung ausbilden kann, können die Volumenangaben in einem solchen Fall auch pro Beschichtung (enthaltend eine Lage) verstanden werden.

Gemäß einem weiteren Aspekt der Erfindung wird bei dem oben beschriebenen Verfahren das Besprühen gemäß Schritt b) mindestens zwei Mal ausgeführt, um eine Beschichtung mit mindestens zwei Lagen zu erzeugen. Zwischen dem Besprühen kann eine Trocknungsphase liegen. Diese kann 30 s bis 30 min betragen, bevorzugt 1 min bis 25 min, ganz besonders bevorzugt 2 min bis 20 min und am besten 3 min bis 15 min.

Teil der Erfindung ist auch eine Wundkontaktschicht wie hierin beschrieben, erhältlich oder erhalten durch das zuletzt erläuterte Herstellungsverfahren.

Ein anderes erfindungsgemäßes Verfahren zur Herstellung einer beschichteten antimikrobiellen Wundkontaktschicht umfasst die folgenden Schritte:
a) Bereitstellung einer silikonhaltigen Wundkontaktschicht,
b) Beschichten zumindest einer Seite der bereitgestellten Wundkontaktschicht durch Auftragen von mindestens zwei übereinanderliegenden Lagen, wobei mindestens eine Lage i) eine Hyaluronsäure enthält und eine negative Nettoladung hat und mindestens eine Lage ii) ein Polypeptid enthält und eine positive Nettoladung hat, wobei das Polypeptid ausgewählt ist aus Polyarginin, Polylysin und Polyornithin oder einer Mischung aus mindestens zwei der zuvor genannten Polypeptide und wobei die übereinanderliegenden Lagen in ihrer Nettoladung alternieren
c) optionale ein- oder mehrmalige Wiederholung von Schritt b)
d) optionales Rücktrocknen der beschichteten Wundkontaktschicht.

Bevorzugt wird in Schritt b) zumindest die proximale Seite der Wundkontaktschicht beschichtet. Im Rahmen dieses Verfahrens kann die Methode der Tauchbeschichtung eingesetzt werden.

Die negative Nettoladung der Hyaluronsäure enthaltenden Lage wird durch die negativ geladene Hyaluronsäure vermittelt und die positive Nettoladung der Polypeptid enthaltenden Lage wird durch die positiv geladenen Aminosäuren des Polypeptids vermittelt.

Empfohlen wird sowohl die Hyaluronsäure als auch das Polypeptid oder die Polypeptide vor dem Beschichten in Lösung zu bringen. Geeignete Lösungsmittel und Konzentrationen wurden hierin bereits an anderer Stelle beschrieben und sind im Rahmen des zuletzt genannten Verfahrens anwendbar. So können das Polypeptid oder die Polypeptide beispielsweise mittels einer Lösung aufgebracht werden, in der sie in einer Konzentration von 0,1 mg / mL bis 100 mg / mL enthalten sind und / oder beispielsweise die Hyaluronsäure mittels einer Lösung aufgetragen werden, die die Hyaluronsäure in einer Konzentration von 0,1 bis 10 mg / mL enthält.

Das zuletzt beschriebene Verfahren eignet sich insbesondere zur Herstellung solcher Wundkontaktschichten, deren Beschichtung mindestens zwei nicht durchmischte Lagen enthält. Nicht durchmischte Lagen sind an anderer Stelle hierin in ihrer Beschaffenheit erläutert. In der Regel enthalten die mit dem obigen Herstellungsverfahren erzeugten Beschichtungen mindestens zwei Lagen. Bevorzugt sind sämtliche Lagen der mittels dieses Verfahrens erzeugten Beschichtung nicht durchmischt. Dabei können sämtliche Lagen mittels Tauchbeschichtung erzeugt werden.

Im Rahmen des zuletzt genannten Verfahrens ist es möglich mittels Tauchbeschichtung eine Vielzahl von Lagen zu einer Beschichtung zu vereinen. Empfohlen wird im Rahmen von Schritt b) nach dem Auftragen einer Lage diese mit einer Pufferlösung zu spülen. Dies sollte erst dann geschehen, wenn die zu spülende Lage ausreichend getrocknet oder angetrocknet ist, um ein ungewolltes Abwaschen zu vermeiden.

Geeignete Pufferlösungen und pH-Werte sind hierin an anderer Stelle genannt. Bevorzugt wird hierzu wässrige Pufferlösung von Tris-NaCl verwendet. Besonders bevorzugt sind sowohl Tris als auch NaCL in einer Konzentration von jeweils 10 mmol bis 150 mmol in der Pufferlösung vorhanden. Die Konzentrationen von Tris und NaCl müssen dabei nicht identisch sein.

Der Beschichtungsschritt b) kann insbesondere mittels Tauchverfahren ausgeführt werden. Wenn das Verfahren mithilfe der Tauchbeschichtung durchgeführt wird, wird in der Regel die Wundkontaktschicht beidseitig (distale und proximale Seite) vollflächig beschichtet. Andere Beschichtungstechniken sind ebenfalls möglich. So kann das Beschichten beispielsweise alternativ auch durch Auftragung mittels Walze oder durch Bestreichen (z.B. mittels Pinsel) erfolgen. Wird Schritt b) des zuletzt genannten Verfahrens mithilfe der Methode der Tauchbeschichtung durchgeführt, erfolgt bevorzugt ein (vollständiges oder teilweises) Eintauchen der Wundkontaktschicht in eine Lösung, die das Polypeptid enthält und / oder in eine Lösung, die die Hyaluronsäure enthält. Wichtig ist, dass jede der zur Tauchbeschichtung verwendeten Lösungen nur entweder die Hyaluronsäure oder das Polypeptid enthält, aber nicht beide gleichzeitig.

Das (vollständige oder teilweise) Eintauchen kann dabei für die Erzeugung einer einzelnen Lage über eine Zeitspanne von ein bis zehn Minuten erfolgen. Das heißt, dass die Wundkontaktschicht als Ganzes in die Lösung eingetaucht wird und nach ein bis zehn Minuten entnommen wird. Die folgenden alternativen Zeitspannen sind ebenfalls möglich: zwei bis neun Minuten, drei bis acht Minuten sowie vier bis sieben Minuten.

Teil des zuletzt genannten Verfahrens ist darüber hinaus, dass Schritt b) wiederholt stattfinden kann (also mindestens zwei Mal). So kann Schritt b) für die Herstellung einer erfindungsgemäßen Wundkontaktschicht beispielsweise zehn bis hundert Mal wiederholt werden, so dass 20 bis 200 Lagen übereinanderliegend aufgetragen werden. Dies resultiert folglich in einer Wundkontaktschicht mit einer Beschichtung, die 20 bis 200 Lagen enthält. Andere mögliche Wiederholungsanzahlen für Schritt b) sind 15 bis 90 Mal, 20 bis 80 Mal, 25 bis 70 Mal und 30 bis 60 Mal.

Wenn die Wundkontaktschicht gemäß Schritt b) mittels Tauchverfahren ausgeführt wird, beziehen sich die eben genannten Wiederholungszahlen auf die Anzahl des Eintauchens in eine Lösung enthaltend die Hyaluronsäure oder das Polypeptid.

Teil der Erfindung ist auch eine beschichtete Wundkontaktschicht wie hierin beschrieben, erhältlich oder erhalten durch das zuletzt erläuterte Herstellungsverfahren unter Einsatz von Tauchbeschichtung.

Die hierin beschriebenen Herstellungsverfahren können sich bei Bedarf durch folgende Punkte auszeichnen:
- In der zu beschichtenden silikonhaltigen Wundkontaktschicht können diejenigen Materialien (z.B. Substrate) enthalten sein, die hierin für die bereits beschichtete Wundkontaktschicht beschrieben sind. Sofern es sich um ein zu beschichtendes Laminat handelt, welches an seiner distalen Außenseite ein Adhäsiv (z.B. auf Akrylbasis) tragen soll, so kann dieses Adhäsiv vor oder nach der Ausstattung mit der antimikrobiellen Beschichtung appliziert werden.
- Die zur Beschichtung bereitgestellte Wundkontaktschicht kann zunächst einer Plasmareinigung unterzogen werden. Die Plasmareinigung findet vor der Beschichtung (z.B. dem Besprühen) statt und kann unter Umständen die Anhaftung der antimikrobiellen Beschichtung verbessern.
- Die hierin beschriebenen Verfahren können automatisiert sein. Bevorzugt findet die Automatisierung mittels eines Roboters statt. Bei dem Roboter kann es sich um einen programmierbaren Roboter handeln. Der Roboter kann über mindestens einen beweglichen Arm verfügen, an dem ein oder mehrere Wundkontaktschichten angebracht werden können. Insbesondere das Auftragen der Beschichtung mittels Tauchverfahren profitiert von einer Automatisierung, da diese Methode in der Regel mehr Zeit beansprucht als das Auftragen der Beschichtung mittels Sprühverfahren. Bevorzugt werden bei dem automatisierten Tauchverfahren Beschichtungen mit mindestens 20, besser 25 und am besten 30 Lagen auf der Wundkontaktschicht erzeugt. Wenn das erfindungsgemäße Herstellungsverfahren mittels eines Roboters stattfindet, können mehrere Wundkontaktschichten gleichzeitig beschichtet werden. Beispielsweise können zwei, mindestens zwei, drei, mindestens drei, vier oder mindestens vier, fünf oder mindestens fünf Wundkontaktschichten auf diese Weise beschichtet werden. Besonders bevorzugt werden zwei bis hundert, zwei bis fünfzig oder zwei bis zehn Wundkontaktschichten gleichzeitig unter Einsatz eines einzigen Roboters zeitgleich beschichtet.

Das Sprühverfahren profitiert ebenfalls von einer Automatisierung, da hierdurch ein gleichbleibender Abstand zwischen Sprühdüse und Wundkontaktschicht während des Sprühvorgangs gewährleistet wird, was bei einem händischen Ansatz deutlich schwieriger umzusetzen ist.

Darüber hinaus umfasst die Erfindung auch ein Kit umfassend
a) eine erfindungsgemäße antimikrobiell beschichtete Wundkontaktschicht und
b) ein Befestigungsmittel, wobei das Befestigungsmittel geeignet ist zur Befestigung der Wundkontaktschicht über einer Wunde. Dazu kann das Befestigungsmittel beispielsweise über Klebemittel (z.B. einen Adhäsivauftrag) verfügen oder geeignet sein über die distale Abschlussschicht der Wunauflage (z.B. Vliesmaterial oder Backing) sowie den betreffenden Körperbereich gewickelt zu werden. Bei dem Befestigungsmittel kann es sich um eine Klebefolie handeln. Bei dem Kit kann es sich um eine Verpackung oder ein Set handeln.

Daneben betrifft die Erfindung auch die Verwendung der erfindungsgemäßen Wundkontaktschicht zur Herstellung einer absorbierenden Wundauflage.

Ein weiterer Aspekt der Erfindung betrifft die erfindungsgemäße Wundkontaktschicht zur Anwendung in einem Verfahren zur Behandlung von Wunden, bevorzugt von infizierten Wunden, besonders bevorzugt von Wunden, die mit S. *aureus* und / oder *P. aeruginosa* infiziert sind.

Dabei kann vorgesehen sein, dass die Anwendung über einen Zeitraum von 0 bis 24 Stunden stattfindet oder, dass die Anwendung in einem Zeitraum von mindestens 24 Stunden stattfindet, wobei letzteres beispielswiese bei bereits stark ausgeprägten Infektionen oder bei Patienten mit Immunschwäche geboten sein kann.

Die erfindungsgemäße Wundkontaktschicht ist geeignet zur Abdeckung und / oder Therapie von Wunden, insbesondere infizierten Wunden. Aus diesen Gründen kann die Wundkontaktschicht eingesetzt oder verwendet werden in einem Verfahren zur Wundtherapie und / oder zur Reduktion der Keimzahl in Wunden und / oder zur Prävention von Wundinfektionen. Bei den Wunden kann es sich insbesondere um traumatische Wunden (inklusive Schnitt- und Operationswunden), chronische Wunden, blutende Wunden, eiternde Wunden und exsudierende (nässende) Wunden handeln. Die erfindungsgemäße Wundkontaktschicht kann auch im Rahmen der Kompressionstherapie unter Kompressionsbandagen oder Kompressionsstrümpfen eingesetzt und von Patienten getragen werden.

### Figuren

Im Folgenden werden die Figuren näher erläutert.

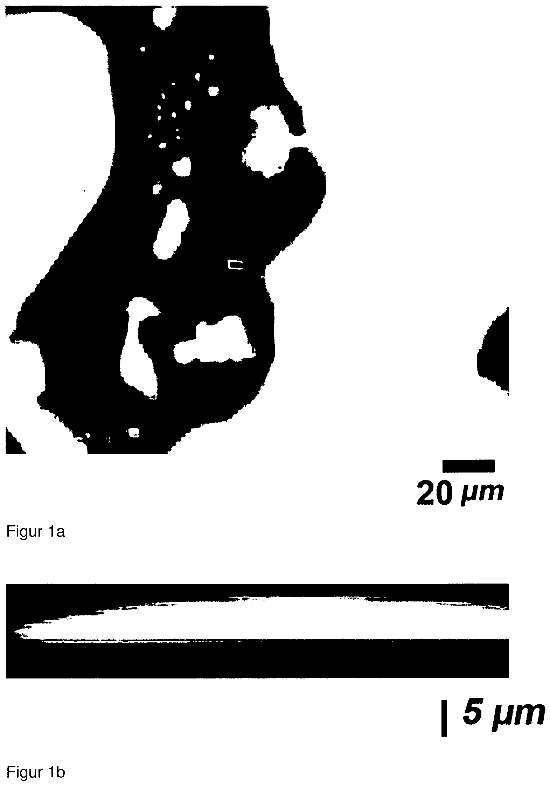

Fig. 1a zeigt die Draufsicht einer Fluoreszenzaufnahme einer Silikonwundkontaktschicht mit antimikrobieller Beschichtung enthaltend eine durchmische Lage mit Hyaluronsäure und Polyarginin. Die Beschichtung wurde durch Besprühen der Silikonwundkontaktschicht aufgetragen. Die Aufnahme wurde mittels Konfokalmikroskop erstellt, wobei das in der Beschichtung enthaltene Polyarginin mittels des fluoreszierenden Markers FITC sichtbar gemacht wurde. Der Größenmaßstab ist in der Darstellung angegeben. schwarz = Silikon ; weiß = Beschichtung
Fig. 1b zeigt dieselbe Silikonwundkontaktschicht wie Fig. 1a allerdings in diesem Fall in der Seitenansicht.

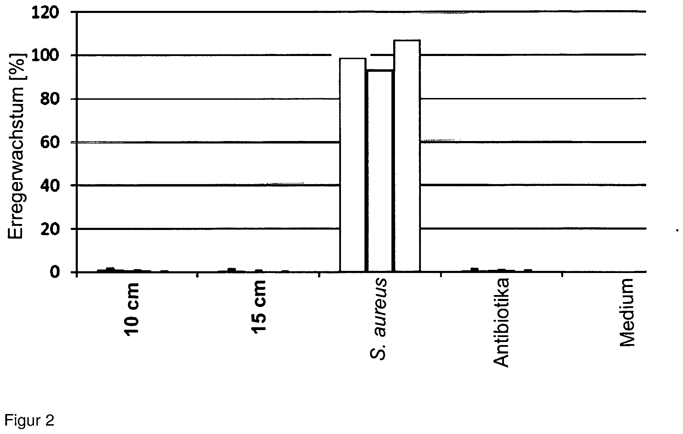

Fig. 2 zeigt die antibakterielle Wirkung von sprühbeschichteten Wundkontaktschichten aus Silikon gegenüber S. *aureus* nach 24 h. Die Beschichtung enthielt Polylysin und Hyaluronsäure in einer durchmischten Lage und wurde aus einer Entfernung von entweder 10 oder 15 cm aufgesprüht. Als Positivkontrolle diente eine Erregersuspension mit Antibiotika. Als Negativkontrolle diente eine Erregersuspension, die nicht einer antibakteriellen Beschichtung ausgesetzt wurde. Um eine Kontamination des Medium auszuschließen, wurde ungeimpftes Medium (ohne Erreger) ebenfalls getestet. Das Erregerwachstum ist als prozentualer Zuwachs gegenüber dem Startzeitpunkt angegeben.

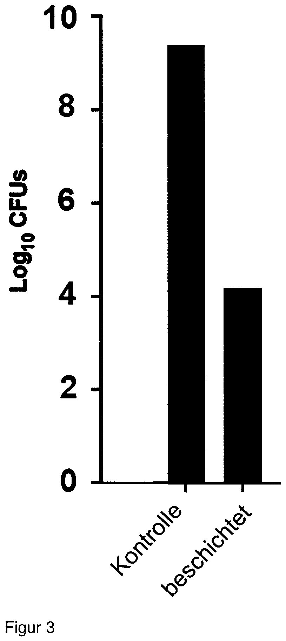

Fig. 3 zeigt die antibakterielle Wirkung von sprühbeschichteten Wundkontaktschichten aus Silikon gegenüber *P. aeruginosa* im Vergleich zu einer unbeschichteten Kontrollschicht. Die Beschichtung enthielt Polylysin und Hyaluronsäure in einer durchmischten Lage. Die Darstellung der Keimzahlen erfolgt anhand eines dekadisch-logarithmischen Maßstabs der Ordinate.

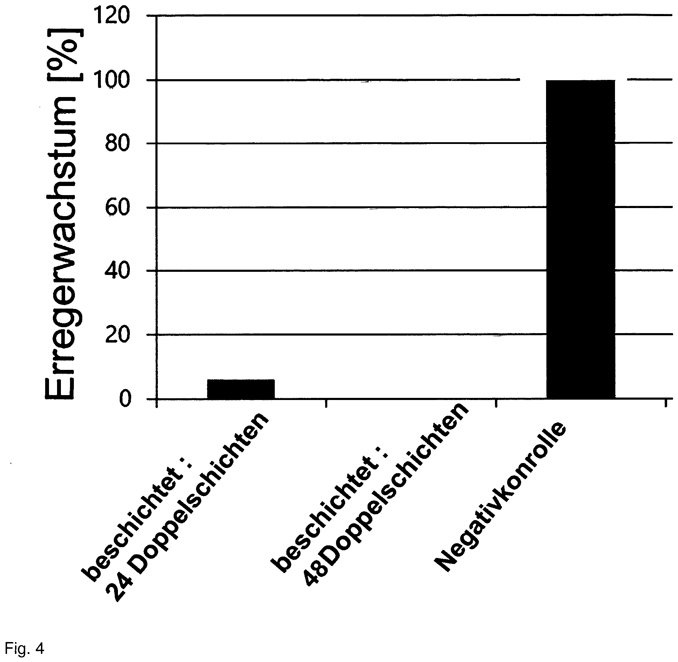

Fig. 4 zeigt die antimikrobielle Wirksamkeit von tauchbeschichteten Wundkontaktschichten mit entweder 24 oder 48 Doppelschichten aus Polyarinin und Hyaluronsäure gegenüber S. *aureus.* Als Negativkontrolle diente eine baugliche Wundkontaktschicht ohne Beschichtung. Bestimmt wurde das Erregerwachstum nach einer Kontaktzeit von 24 h.

### Beispiele

### Beispiel 1: Materialien

An Materialien wurden bereitgestellt:
- Polypeptide vom Typ Polyarginin als synthetisches Polymer bestehend aus 30 Aminosäuren pro Molekül ("PAR30"), so dass jedes Molekül eine Molekülmasse von etwa 5,8 kDa hatte. Das Polyarginin wurde vom Unternehmen "AlamandaTM Polymers" bezogen. Die eingesetzte Konzentration lag bei 0,5 mg / ml.
- Polypeptide vom Typ Polylysin, welche als ε-Poly(L-Lysin) natürlichen Ursprungs (Herstellung mittels Bakterien aus der Familie der Streptomycetaceae) vom Unternehmen Biosynth^{®} bezogen wurden. Die mittlere Molekülmasse lag zwischen 3,5 und 4,5 kDa. Die eingesetzte Konzentration lag bei 10 mg / ml Tris-NaCl-Puffer.
- Hyaluronsäure aus 144 repetitiven Untereinheiten pro Molekül ("HA144"). Hergestellt mittels rekombinanter Produktion aus Mikroorganismen. Die eingesetzte Konzentration lag bei 0,5 mg / ml in Tris-NaCl-Puffer. Bezogen wurde die Hyaluronsäure vom Unternehmen Lifecore^{®} Biomedical.
- Zwei verschiedene silikonhaltige Wundkontaktschichten:
   A): Eine Wundkontaktschicht aus Silikon in medizinischer Qualität B) Eine adhäsive, atraumatische Wundkontaktschicht in Form eines perforierten Laminats. Dabei war eine PU-Folie wundseitig bedeckt mit einer Schicht aus adhäsivem Silikongel und auf der wundabgewandten Seite mit einem Adhäsiv auf Acrylbasis. Das Adhäsiv auf Acrylbasis wurde mit einer Schutzfolie abgedeckt.

### Beispiel 2a: Auftrag einer polylysinhaltigen Beschichtung mittels Sprühverfahren

Als Ausgangsmaterial diente die in Beispiel 1 beschriebene Wundkontaktschicht "A". Es wurden vier Prüfmuster mit einer Fläche von jeweils 2,25 cm² bereitgestellt. Bei der Beschichtung mittels Sprühverfahren kamen eine Lösung enthaltend ε-Poly(L-Lysin) (10 mg / ml) und eine Lösung enthaltend Hyaluronsäure ("HA144" ; 0,5 mg / ml) zum Einsatz. Als Lösungsmittel diente Tris-NaCl-Puffer. Beide Lösungen wurden zeitgleich über jeweils eine separate Düse einer Sprühpistole auf die wundzugewandte Seite der Wundkontaktschicht (proximale Seite) aufgesprüht. Das Aufsprühen fand aus einer Distanz von 10 cm oder 15 cm statt. Dabei wurde die Sprühpistole während des Sprühens zehn Mal für jeweils eine Sekunde über jedes Prüfmuster geführt, um pro Muster insgesamt 1,5 ml von jeder Lösung aufzutragen. Somit Betrug das Flächenvolumen der Beschichtungslösung 0,66 ml / cm² Wundkontaktfläche. Der Beschichtungsvorgang dauerte 10 - 15 Sekunden. Anschließend folgte eine Trocknung (über Nacht, passiv bei Raumtemperatur).

### Beispiel 2b: Auftrag einer polyargininhaltigen Beschichtung mittels Sprühverfahren

Das Vorgehen war analog zu Beispiel 2a. Als Lösungen kamen zum Einsatz:
a) 1 mg / ml Polyarginin in Kombination mit 1 mg / ml Hyaluronsäure sowie
b) 2 mg / ml Polyarginin in Kombination mit 1 mg / ml Hyaluronsäure.

### Beispiel 3: Visuelle Inspektion der Sprühbeschichtung mittels Konfokalmikroskop

Die im Sprühverfahren gemäß Beispiel 2b erzeugte Beschichtung wurde einer visuellen Prüfung mittels Konfokalmikroskop unterzogen. Hierzu wurde das in der Beschichtung enthaltene Polyarginin mit dem Fluorophor Fluoresceinisothiocyanat (FITC) markiert (PAR30-FITC).

Die Verteilung der auf diese Weise zur Fluoreszenz gebrachten Moleküle wurde mit einem Mikroskop vom Typ Zeiss LSM 710 (Konfokales Laserscanning Mikroskop) begutachtet. Dies fand unter einer 40-fachen Vergrößerung statt. Die korrekte Anhaftung der Beschichtung an der Wundkontaktschicht aus Silikon konnte verifiziert werden. Das Ergebnis ist in Fig. 1a (Draufsicht) und Fig. 1b (Seitenansicht) als Fotoaufnahme dargestellt. Helle bzw. weiße Bereiche repräsentieren die Fluoreszenz. Schwarze Bereiche repräsentieren die Wundkontaktschicht.

Wie zu erkennen ist, wurde ein von der antimikrobiellen Beschichtung ausgehendes Fluoreszenzsignal generiert. Dies lässt auf eine erfolgreiche Anhaftung der Beschichtung am Silikon schließen.

### Beispiel 4: Antimikrobielle Wirksamkeit einer polylysinhaltigen Sprühbeschichtung gegenüber S. aureus

Für den Test eingesetzt wurden die in Beispiel 2a mittels Sprühverfahren beschichteten Wundkontaktschichten. Diese waren ausgestattet mit einer Sprühbeschichtung bestehend aus einer durchmischten Lage enthaltend ε-Poly(L-Lysin) (10 mg / ml) und HA144 (0.5mg / ml). Die antimikrobielle Wirksamkeit der mittels Spühbeschichtung ausgestatteten Träger wurde mithilfe einer verkürzten Variante des Standards ISO 20743:2021 getestet (Owen L, Laird K. Development of a silver-based dual-function antimicrobial laundry additive and textile coating for the decontamination of healthcare laundry. J Appl Microbiol. 2021 ;130(4):1012-22). Der Test fand gegenüber *Staphylococcus aureus* (Hinterlegung ATTC 25923) statt. Die Messung wurde insgesamt drei Mal durchgeführt und die Mittelwerte gebildet. Dabei wurde die Zunahme der Erregeranzahl nach einer Kontaktzeit von 24 h ermittelt. Die Ergebnisse sind in Fig. 2 zu sehen, wobei jeder Balken des Diagramms den Mittelwert aus drei Messungen repräsentiert. Als Positivkontrolle wurden Antibiotika zum Kulturmedium hinzugefügt. Als Negativkontrolle diente eine Suspension von S. *aureus* ohne Kontakt zur antimikrobiellen Beschichtung und ohne Antibiotika. Daneben wurde auch das unbeimpfte Kulturmedium ohne Erreger vermessen, um mögliche Fehlerquellen wie eine unbeabsichtigte Kontamination auszuschließen. Wie aus den Darstellungen deutlich wird, war ein ausgeprägter antimikrobieller Effekt nachweisbar und zwar sowohl für eine Beschichtung, die aus 10 cm aufgesprüht wurde als auch für eine Beschichtung, die aus 15 cm aufgesprüht wurde. Die antimikrobielle Wirksamkeit war derjenigen konventioneller Antibiotika (Positivkontrolle) ebenbürtig. Im unbeimpfte Medium konnte wie erwartet keine Vermehrung von Erregern festgestellt werden.

### Beispiel 5: Antimikrobielle Wirksamkeit einer polylysinhaltigen Sprühbeschichtung gegenüber P. aeruginosa

Für den Test eingesetzt wurden die in Beispiel 2a mittels Sprühverfahren beschichteten Wundkontaktschichten. Die antimikrobielle Wirksamkeit der mittels Spühbeschichtung ausgestatteten Träger wurde gemäß ISO 20743:2021 getestet. Der Test fand gegenüber *Pseudomonas aeruginosa* (aus Hinterlegung ATTC 27853) statt. Die Messung wurde insgesamt drei Mal durchgeführt und die Mittelwerte gebildet. Beim Test wurde die Reduktion der Anzahl teilungsfähiger Bakterienzellen (CFU) auf den Wundkontaktschichten nach einer Kontaktzeit von 24 h ermittelt.

Zunächst wurden die beschichteten Wundkontaktschichten mit einer Bakterienausgangskonzentration von 1 bis 3 × 10⁵ CFU / ml beimpft und bei 37 °C inkubiert. Im Anschluss (nach 24 h) wurden die überlebenden Bakterien in PBS eluiert. Das Eluat wurde abwechselnd gevortext (mit hoher Frequenz mittels eines Vortex Gerätes geschüttelt), sonifiziert und wieder gevortext (jeweils 30 Sek. in dreifacher Wiederholung). Als Kontrolle dienten unbeschichtete Wundkontaktschichten mit ansonsten identischem Aufbau. Die Tests wurden mit drei biologischen und drei technischen Wiederholungen durchgeführt. Die ermittelte antimikrobielle Aktivität ist ausgedrückt als Mittelwert der logarithmischen Anzahl vermehrungsfähiger Bakterien. Die Ergebnisse sind in Fig. 3 dargestellt. Wie der Abbildung entnommen werden kann, wurde eine Keimreduktion von ca. ≥ 5 iog10 gegenüber *P. aeruginosa* im Vergleich zur Negativkontrolle erreicht.

Wie aus den Darstellungen deutlich wird, war ein ausgeprägter antimikrobieller Effekt nachweisbar.

### Beispiel 6a: Messung der Abzugskraft einer antimikrobiell beschichteten Wundkontaktschicht

Die Messung der Abzugskraft fand als sogenannter Schälversuch statt und ermittelte die Kraft, welche für das Abziehen der Wundkontaktschichten (analog zu einem Verbandswechsel) notwendig war.

Die Messung fand an einer antimikrobiell beschichteten Wundkontaktschicht vom Typ "B" (siehe Beispiel 1) ohne Schutzfolie statt. Die antimikrobielle Beschichtung war zuvor analog zu Beispiel 2a mittels Sprühverfahren aufgetragen worden.

Die Prüfmuster hatten eine Länge von 2 cm und eine Breite von 2,5 cm. Zur Durchführung der Messung wurden die Prüflinge mittels eines konventionellen Klebebands (Typ 4104 der Firma Tesa - Klebeband ohne Eigendehnung) verlängert, um ein Einspannen in die Messvorrichtung zu ermöglichen.

Als Messvorrichtung diente eine Zugprüfmaschine vom Typ "Autograph AGX-V 10kNVD" mit 1kN Messdose von der Firma Shimadzu. Die Prüfmuster wurden für die Messung mit der Wundkontaktseite voran auf eine Stahlplatte (entsprach DIN EN 1939:2003-12) mittels Anrollen (20N/cm ; 150cm/min) appliziert. Eine Minute nach Abschluss des Anrollens wurden die Prüfmuster mithilfe der Zugprüfmaschine in einem Abzugswinkel von 90° abgezogen. Die Zuggeschwindigkeit betrug 30 cm / min. Als Vergleichsreferenz diente eine baugleiche Wundkontaktschicht, die nicht mit einer antimikrobiellen Beschichtung versehen worden war, wobei die Referenzkontaktschicht mit dem adhäsiven Silikongel (entsprechend der Wundkontaktfläche) voran auf die Stahlplatte appliziert wurde. Vermessen wurden zwei erfindungsgemäße Wundkontaktschichten und zwei Referenzkontaktschichten. Die Auswertung des von der Zugprüfmaschine ausgegebenen Kurvenverlaufs erfolgte gemäß dem Standard DIN ISO 6133:2004-05 unter Einbezug von neun Kraftspitzen innerhalb des Kurvenverlaufs.

Die Ergebnisse sind in Tab. 1 dargestellt:

**Tab. 1: Ermittelte Abzugskraft**

| Probennummer | Antimikrobiell beschichtete Wundkontaktschicht [Fₘₐₓ in N / 25 mm] | Unbeschichtete Referenzkontaktschicht [Fₘₐₓ in N / 25 mm] |
|---|---|---|
| 1 | 0,99 | 0,88 |
| 2 | 1,03 | 0,61 |
| Durchschnittswert | 1,01 | 0,75 |

Wie der Vergleich zeigt, liegt die für den Abzug notwendige Kraft bei der antimikrobiell beschichteten Wundkontaktschicht im selben Bereich, wie bei der unbeschichteten Referenzkontaktschicht. Es kann festgestellt werden, dass die atraumatischen Eigenschaften der Wundkontaktschicht auch nach Auftragen der antimikrobiellen Beschichtung erhalten bleiben, so dass ein schonender sowie schmerzloser Verbandswechsel weiterhin möglich ist.

### Beispiel 6b: Messung der Klebekraft einer antimikrobiell beschichteten Wundkontaktschicht

Ziel der Messung war es die Haftung der Prüfmuster auf einer Unterlage (hier Glasfläche) zu bestimmen. Je höher die Klebekraft, desto besser die Haftung und desto geringer die Wahrscheinlichkeit, dass sich die Wundkontaktfläche unbeabsichtigterweise von der Haut löst. Dazu wurde die zum Abzug notwendige Kraft bestimmt. Im Unterschied zur Messung gemäß Beispiel 6a wurden die Prüfmuster nicht in einem 90° Winkel abgezogen, sondern die Prüfmuster bleiben während des gesamten Versuchs plan an einer Stahlplatte.

Die Messung bestimmte die Anfangshaftkraft der Prüfmuster (sogenannter Tacktest) und fand an einer antimikrobiell beschichteten Wundkontaktschicht vom Typ "B" (siehe Beispiel 1) ohne Schutzfolie statt. Die antimikrobielle Beschichtung war zuvor analog zu Beispiel 2a mittels Sprühverfahren aufgetragen worden. Als Messvorrichtung diente eine Zugprüfmaschine vom Typ "Autograph AGX-V 10kNVD" mit 1kN Messdose von der Firma Shimadzu. Um ein unbeabsichtigtes Anheben der Prüfmuster während der Messung zu verhindern, werden diese mit der distalen (wundabgewandten) Seite voran mittels eines doppelseitig klebenden Klebebands auf einer Stahlplatte fixiert. Während des Tests wurde ein Zuggewicht aus Metall mit einer planaren Kontaktfläche aus rauem Glas (0,245 N) von der adhäsiven Seite (Wundkontaktseite) der Prüfmuster abgezogen und die dazu notwendige Kraft von der Zugprüfmaschine erfasst. Die Anfahrgeschwindigkeit betrug 10 cm / min, die Kontaktzeit 2 s.

Als Vergleichsreferenz diente eine baugleiche Wundkontaktschicht, die nicht mit einer antimikrobiellen Beschichtung versehen worden war, wobei die Referenzkontaktschicht mit dem adhäsiven Silikongel (entsprechend der Wundkontaktfläche) voran mit dem Zuggewicht in Kontakt gebracht wurde. Vermessen wurden zwei erfindungsgemäße Wundkontaktschichten und zwei Referenzkontaktschichten.

Die Ergebnisse sind in Tab. 2 dargestellt:

**Tab. 2: Ermittelte Klebekraft**

| Probennummer | Antimikrobiell beschichtete Wundkontaktschicht [N] | Unbeschichtete Referenzkontaktschicht [N] |
|---|---|---|
| 1 | 3,6 | 3,8 |
| 2 | 5,8 | 4,0 |
| Durchschnittswert | 4,7 | 3,9 |

Wie aus den gemessenen Werten ersichtlich ist, bewegt sich die ermittelte Klebekraft der antimikrobiell beschichteten Wundkontaktschicht im selben Bereich wie diejenige der unbeschichteten Referenzkontaktschicht und übersteigt diese sogar geringfügig. Somit kann festgestellt werden, dass die getesteten Wundkontaktschichten nach Auftrag der antimikrobiellen Beschichtung mindestens genauso gut haften, wie Referenzkontaktschichten ohne die antimikrobielle Beschichtung.

### Beispiel 7: Beschichtung mittels Tauchverfahren

Als Ausgangsmaterial dienten Wundkontaktschichten des Typs "A" wie unter Beispiel 1 beschrieben. Die Wundkontaktschicht wurde zunächst in einem Autoklaven sterilisiert. Während des nachfolgenden Beschichtungsvorgangs mittels Tauchverfahren wurde der Vliesstoff abwechselnd in ein Bad mit Polyarginin ("PAR30" ; 0,5 mg / mL) und ein Bad mit Hyaluronsäure (0,5 mg / mL) eingetaucht. Der erste Eintauchschritt erfolgte dabei in der Lösung mit Polyarginin. Das Eintauchen erfolgte jeweils für 200 Sekunden. Nach jedem Tauchschritt wurden die Wundkontaktschichten gespült, und zwar ebenfalls für 200 Sekunden. Hierzu wurde Tris-NaCl-Puffer verwendet (10 mmol Tris, 150 mmol NaCl, pH 7,4). Der Prozess fand voll automatisiert statt unter Einsatz eines Roboters der Firma Riegler & Kirstein GmbH.

Die genannten Schritte wurden so oft wiederholt, bis entweder 24 oder 48 Doppellagen auf das Substrat aufgetragen waren. Jede Doppellage bestand aus einer Lage enthaltend das Polyarginin und einer Lage enthaltend die Hyaluronsäure. Es folgte eine Trocknung über Nacht (passiv bei Raumtemperatur.

Die erfolgreiche Beschichtung konnte wie in Beispiel 3 angegeben nach Markierung mittels des Fluoreszenzmarker FITC mittels Konfokalmikroskop bestätigt werden (nicht dargestellt).

### Beispiel 8: Antimikrobielle Wirksamkeit von tauchbeschichteten Wundkontaktschichten

Die antimikrobielle Wirksamkeit der in Beispiel 7 mit einer Tauchbeschichtung ausgestatteten Wundkontaktschichten wurden mithilfe einer verkürzten Variante des Standards ISO 20743:2021 auf ihre antimikrobielle Wirkung getestet. Der Test fand gegenüber *S*. *aureus* (Hinterlegung ATTC 25923) statt. Als Negativkontrolle diente eine baugliche Wundkontaktschicht ohne Beschichtung. Bestimmt wurde das Erregerwachstum nach einer Kontaktzeit von 24 h mit den Wundkontaktschichten. Die Ergebnisse sind in Fig. dargestellt. Wie zu erkennen ist, zeigen sowohl die Wundkontaktschichten mit 24 Doppellagen als auch diejenigen mit 48 Doppellagen eine ausgeprägte antimikrobielle Wirkung im Vergleich zur Negativkontrolle.

## Patentansprüche

1. Silikonhaltige, antimikrobielle Wundkontaktschicht, die wundseitig eine teilweise oder vollständige antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst.

2. Silikonhaltige, antimikrobielle Wundkontaktschicht gemäß Anspruch 1, wobei das Polypeptid mindestens zehn Aminosäuren und/oder höchstens hundert Aminosäuren umfasst.

3. Silikonhaltige, antimikrobielle Wundkontaktschicht gemäß Anspruch 1 oder 2, wobei die Hyaluronsäure als Polymermischung mit unterschiedlicher Kettenlänge vorliegt und wobei die Polymermischung Polymere der Hyaluronsäure mit einem Molekulargewicht von mindestens 10 kDa und / oder höchstens 300 kDa umfasst.

4. Silikonhaltige, antimikrobielle Wundkontaktschicht nach einem der vorhergehenden Ansprüche, wobei die Beschichtung mindestens zwei übereinanderliegende und miteinander verbundenen Lagen enthält, und wobei mindestens eine Lage vorhanden ist, welche das Polypeptid enthält und eine positive Nettoladung hat, sowie mindestens eine Lage, welche die Hyaluronsäure enthält und eine negative Nettoladung hat, und wobei sich bei den übereinanderliegenden Lagen jeweils eine das Polypeptid enthaltende Lage mit einer die Hyaluronsäure enthaltenden Lage abwechselt, so dass eine Abfolge von alternierenden Lagen gebildet wird.

5. Silikonhaltige, antimikrobielle Wundkontaktschicht nach Anspruch 4, wobei die Anzahl der übereinanderliegenden und miteinander verbundenen Lagen in der Beschichtung 10 bis 100 beträgt.

6. Silikonhaltige, antimikrobielle Wundkontaktschicht nach einem der Ansprüche 1 bis 3, wobei das Polypeptid und die Hyaluronsäure innerhalb der Beschichtung oder innerhalb mindestens einer Lage der Beschichtung durchmischt vorliegen und das Polypeptid in einer Hyaluronsäurematrix eingebettet ist.

7. Silikonhaltige, antimikrobielle Wundkontaktschicht nach einem der vorhergehenden Ansprüche, wobei das Polypeptid ein Molekulargewicht von 1 bis 41 kDa hat.

8. Silikonhaltige, antimikrobielle Wundkontaktschicht nach einem der vorhergehenden Ansprüche, wobei die Beschichtung eine Dicke von 10 nm bis 1000 nm hat.

9. Silikonhaltige, antimikrobielle Wundkontaktschicht nach einem der vorhergehenden Ansprüche, wobei die Wundkontaktschicht Öffnungen aufweist, welche den Durchtritt von Wundflüssigkeit erlauben.

10. Silikonhaltige, antimikrobielle Wundkontaktschicht nach einem der vorhergehenden Ansprüche, wobei es sich bei dem Silikon um einen adhäsives Silikongel handelt.

11. Silikonhaltige, antimikrobielle Wundkontaktschicht nach einem der vorhergehenden Ansprüche, wobei die Wundkontaktschicht an ihrer wundabgewandten Seite eine Folie enthält.

12. Silikonhaltige, antimikrobielle Wundkontaktschicht nach Anspruch 11, wobei die Folie an ihrer wundabgewandten Seite eine Klebebeschichtung aufweist.

13. Absorbierende Wundauflage umfassend eine silikonhaltige, antimikrobielle Wundkontaktschicht nach einem der vorhergehenden Ansprüche sowie weiterhin eine Absorptionsschicht.

14. Absorbierende Wundauflage nach Anspruch 13, umfassend ein Backing als Abschlussschicht, wobei das Backing über einen zumindest um die Absorptionsschicht umlaufenden wundseitigen Kleberand verfügt.

15. Verfahren zur Herstellung einer silikonhaltigen, antimikrobiellen Wundkontaktschicht nach einem der Ansprüche 1 bis 12 umfassend die folgenden Schritte:
a) Bereitstellen einer silikonhaltigen Wundkontaktschicht,
b) Besprühen zumindest einer Seite der bereitgestellten Wundkontaktschicht mit i) einer Hyaluronsäure und ii) einem Polypeptid, um eine Beschichtung auszubilden, welche mindestens eine Beschichtungslage umfasst, wobei das Polypeptid ausgewählt ist aus Polyarginin, Polylysin und Polyornithin oder einer Mischung aus mindestens zwei der zuvor genannten Polypeptide und wobei die mindestens eine Beschichtungslage sowohl die Hyaluronsäure als auch das Polypeptid enthält.
